# EUROPÄISCHE PATENTSCHRIFT

(11) **EP 4 017 555 B1**
(45) Veröffentlichungstag und Bekanntmachung des Hinweises auf die Patenterteilung: **27.05.2026**
(21) Anmeldenummer: 20760818.3
(22) Anmeldetag: 20.08.2020
(51) Int. Cl.: A61M 1/36

(54) **DRUCKMESSKAPSELHALTERUNG FÜR EINE EXTRAKORPORALE BLUTBEHANDLUNGSMASCHINE**
PRESSURE-MEASUREMENT CAPSULE HOLDER FOR AN EXTRACORPOREAL BLOOD-TREATMENT MACHINE
SUPPORT POUR CAPSULE DE MESURE DE PRESSION POUR UNE MACHINE DE TRAITEMENT DU SANG EXTRACORPOREL

(30) Priorität: 23.08.2019 DE 102019122705
(43) Veröffentlichungstag der Anmeldung: 29.06.2022
(73) Patentinhaber: B. Braun Avitum AG, 34212 Melsungen (DE)
(72) Erfinder: CORAZZARI, Enrico, 41030 Modena (IT)
(74) Vertreter: Winter, Brandl - Partnerschaft mbB
(86) Internationale Anmeldenummer: PCT/EP2020/073379
(87) Internationale Veröffentlichungsnummer: WO 2021/037687

(56) Entgegenhaltungen:
- EP-A1- 2 233 164
- EP-A2- 1 843 140
- WO-A1-2005/068963
- JP-A- 2010 259 538

## Beschreibung

Die vorliegende Erfindung betrifft eine Druckmesskapselhalterung, welche an einem Gehäuse einer extrakorporalen Blutbehandlungsmaschine, insbesondere einer Dialysemaschine, angebracht oder anbringbar ist, mit einer Greifvorrichtung, die dazu angepasst ist, eine in die Druckmesskapselhalterung eingesetzte Druckmesskapsel um- oder eingreifend zu halten.

### Stand der Technik

Extrakorporale Blutbehandlungsmaschinen, insbesondere Dialysemaschinen, weisen ein Leitungssystem, insbesondere mit einem Dialyseflüssigkeitskreislauf sowie einem extrakorporalen Blutkreislauf, auf. Diese Kreisläufe haben eine Anzahl von Dialyseflüssigkeits- und Blut(schlauch)leitungen, welche diverse Funktionseinheiten der extrakorporalen Blutbehandlungsmaschine, wie z.B. einen Dialysator, Blutpumpen etc., durchlaufen und/oder verbinden. Um eine reibungslose Funktionalität der extrakorporalen Blutbehandlungsmaschine zu gewährleisten, muss dieses Leitungssystem, insbesondere die Blutschlauchleitungen, an mehreren Stellen drucküberwacht werden. Beispielsweise sind bei einer gängigen Dialysemaschine ein PA-Anschluss zur Überwachung des arteriellen Unterdrucks, ein PBE-Anschluss zur Überwachung des Eingangsdrucks vor dem Dialysator und ein PV-Anschluss zur Überwachung des venösen Drucks bereitgestellt.

Zum Zweck der Drucküberwachung werden häufig Druckmesskapseln oder sogenannte PODs (Oszillierenden Druckmembrane) an geeigneten Stellen in den Leitungen oder Blutschlauchleitungen eingebaut, wie beispielsweise bei dem auf dem Markt erhältlichen Medisystems Streamline-System. Entsprechende PODs oder Druckabnehmerkapseln weisen eine volumenstarre Kapsel auf, welche durch eine Membran in zwei Kammern geteilt ist, genauer in eine an ein Blutleitungssystem angeschlossene Blutkammer sowie eine Fluidkammer, genauer Gas- oder Luftkammer, welche über einen dünnen Schlauch oder eine Druckübertragungsleitung mit einem Drucksensor oder einem zugehörigen Anschluss der extrakorporalen Blutbehandlungsmaschine verbunden ist. Entsprechende Anschlüsse sind dabei typischerweise mit Luer-Konnektoren versehen, welche zum Anschließen der Druckübertragungsleitung eine Schraubbetätigung benötigen. Dadurch, dass die Druckabnehmerkapseln in den Schlauchleitungen des Leitungssystems hängen und somit an nicht genau definierten, instabilen Positionen schweben, können Messungenauigkeiten entstehen. Im Betrieb wird ein in dem Leitungssystem und somit in der ersten Kammer anliegender Leitungsinnendruck über die Membran auf die zweite Kammer übertragen, in welcher somit ein von dem Leitungsinnendruck abhängiger Fluiddruck, insbesondere Gasdruck, entsteht. Der mit der zweiten Kammer über den dünnen Schlauch verbundene Drucksensor erhält somit ein Fluiddrucksignal, welches er in ein elektrisches Signal wandelt. Durch den dünnen, flexiblen Schlauch, welcher eine große Fluid- oder Luftstrecke und ein Totvolumen zwischen der Druckabnehmerkapsel und dem Drucksensor bildet, entsteht eine Tiefpasswirkung, sodass nachteiliger Weise die Genauigkeit der Messung und eine Reaktionszeit des Drucksensors eingeschränkt ist.

Alternativ ist aus DE 10 2006 016 846 B4 bekannt, Druckmesskapseln mit einer Luftseite unmittelbar (ohne zwischenliegenden Schlauch) an einen Drucksensor anzuschließen. Des Weiteren offenbaren WO 2014/099767 A1 und US 8 210 049 B2 lösbare Halterungen für eine Druckmesskapsel, welche an einem Gehäuse oder einem permanenten Rahmen angebracht sind. Das heißt, es sind Druckabnehmerkapseln oder PODs bekannt, welche unmittelbar oder über eine volumenstarre, kurze Verbindung mit dem Drucksensor verbunden ist. Nachteilig daran ist, dass die darin beispielhaft genannten Verbindungen, wie z.B. eine Luer-Lockverbindung oder eine Bajonett-Verbindung, aufwändig zu montieren und/oder zu demontieren sind und/oder Anforderungen an die Dichtigkeit der Verbindung ggf. nicht erfüllt werden können. Beispielsweise muss ein Luer-Lock-Konnektor geschraubt werden und ist somit montageaufwändig und können ferner durch zu festes Anziehen der Schraubverbindung die einzelnen Luer-Komponenten stark aufeinandergepresst werden und dadurch schwer zu demontieren sein oder gar beschädigt werden. Zusammenfassend können heutige Systeme mit einer Luer-Lock-Verbindung die Zustände offen/diskonnektiert, fest/geschlossen oder locker/leck (nicht korrekt angeschlossen) annehmen.

Aus der WO 2005 / 068 963 A1 ist eine Druckmesskapsel für eine Flüssigkeit bekannt, die mit einem flüssigkeitsführenden Schlauch verbunden werden kann. Die Druckmesskapsel weist eine Luftsäule auf, die mit einer Membran des Schlauchs in Kontakt steht. Der Schlauch und die Druckmesskapsel sind lösbar durch Schnapp- oder Federhaken verbunden, die in eine Nut an der Druckmesskapsel eingreifen. Wenn die Schnapp- oder Federhaken nicht komplett in die Nut eingreifen, kann sich ein Zustand einstellen, bei dem die Druckmesskapsel nicht vollständig mit dem Schlauch verbunden und somit nicht komplett dicht ist.

Die EP 1 843 140 A2 offenbart eine zweigeteilte Druckmesskapsel. Ein Druckaufnehmer ist dabei durch eine Schraubverbindung lösbar mit einem Fluidleitsystem verbunden. Das Fluidleitsystem nimmt das Blut und der Druckaufnehmer ein Fluid auf. Das Blut und das Fluid sind durch eine Membran voneinander getrennt. Durch die Trennung von Blut und Fluid kann das Fluidleitsystem leichter ausgetauscht werden.

Bei den bekannten Lösungen kann ein Zustand hergestellt werden, bei dem der Nutzer denkt, dass die beiden Teile verbunden sind. Dabei sind die beiden Teile nur unzureichend verbunden. Als Folge kann die Druckmessung fehlerhaft sein oder möglicherweise kann auch Flüssigkeit aus der Verbindung austreten, ohne dass es der Nutzer merkt.

### Zusammenfassung der Erfindung

Die der vorliegenden Erfindung zugrundeliegende Aufgabe besteht darin, Nachteile des Stands der Technik zu verbessern oder zu beseitigen. Insbesondere soll eine Druckmesskapselhalterung bereitgestellt werden, die es ermöglicht, Druckmesskapseln einfach zu montieren und zu demontieren und insbesondere dennoch eine sehr dichte Verbindung zu einer Fluidkammer, insbesondere Luftkammer, einer Druckmesskapsel bereitstellt.

Die der Erfindung zugrundeliegende Aufgabe wird durch eine Druckmessanordnung mit den Merkmalen des Anspruchs 1 gelöst.

Genauer ausgedrückt wird die Aufgabe durch eine Druckmesskapselhalterung gelöst, welche an einem Gehäuse einer extrakorporalen Blutbehandlungsmaschine, insbesondere einer Dialysemaschine, anbringbar ist. Eine Greifvorrichtung, welche dazu angepasst ist, eine in die Druckmesskapselhalterung eingesetzte Druckmesskapsel, die zumindest einen Blutkammeranschluss hat, um- oder eingreifend zu halten, und ein Auswurfmechanismus, welcher dazu angepasst ist, die Druckmesskapsel bei einem Lösen der Greifvorrichtung auszuwerfen, sind bereitgestellt. Ein Innenraum der Druckmesskapsel ist insbesondere durch eine Membran in eine Blutkammer und eine Fluidkammer getrennt. Die Druckmesskapselhalterung hat eine Druckübertragungsleitung, welche dazu konfiguriert ist, an die Fluidkammer der Druckmesskapsel fluidisch anschließbar zu sein und einen in der Fluidkammer anliegenden Fluiddruck an einen Drucksensor der extrakorporalen Blutbehandlungsmaschine zu übertragen. Der Auswurfmechanismus weist einen Federmechanismus auf, mit einem Druckelement oder einem Anpressbauteil, welches derart federnd gelagert ist, dass es bei einem Einsetzen der Druckmesskapsel vorgespannt wird und in einem Haltezustand, in welchem die Druckmesskapsel durch die Greifvorrichtung gehalten wird, gegen diese presst. An einem äußeren Ende des Druckelements ist ein vorzugsweise konisches Halterungsanschlusselement (kurz: Anschlusselement), vorzugsweise aus einem Weichkunststoff, weiter vorzugsweise ein Luer-Konnektor, angeordnet oder ausbildet. Das Halterungsanschlusselement weist die Druckübertragungsleitung auf.

Die Druckübertragungsleitung ist beispielsweise ein Druckübertragungskanal, der an seinem der ggf. eingesetzten Druckmesskapsel zugewandten Leitungs-/Kanalende mit der Fluidkammer verbunden werden kann, insbesondere beim Einsetzen der Kapsel in die Halterung (automatisch / ohne einen zusätzlich zum Einsetzen auszuführenden Montageschritt) mit der Fluidkammer verbunden wird. Darüber hinaus kann der Druckübertragungskanal (an einem der ggf. eingesetzten Druckmesskapsel entgegengesetzten Kanal-/Leitungsende) einen Anschluss aufweisen, der mit einer weiteren (Druckübertragungs-) Leitung oder direkt mit einem Druckaufnehmer (Drucksensor) verbindbar ist.

In anderen Worten ausgedrückt, ist eine Greifvorrichtung oder eine Halteeinrichtung bereitgestellt, die eine Druckmesskapsel (kurz: Kapsel) hält und einen Mechanismus aufweist, welcher bei einem einfachen Herausnehmen der Kapsel aus der Druckmesskapselhalterung (kurz: Halterung) durch einen Nutzer automatisch ein Auswerfen der Kapsel erzielt, d.h., die Kapsel durch eine einfache Betätigung des Nutzers automatisch aus der Halterung derart herausbewegt wird, dass eine erneute einfache Betätigung notwendig ist, um die Kapsel wieder in Eingriff mit der Greifvorrichtung zu bringen. Ggf. wird durch den Auswurfmechanismus ferner eine gasdichte Verbindung zwischen der Kapsel und einer Druckübertragungsleitung/-kanal der extrakorporalen Blutbehandlungsmaschine zumindest teilweise gelöst.

Der Auswurfmechanismus wird zumindest teilweise durch einen Federmechanismus gebildet. In diesem Fall kann die Kapsel entgegen einer Rückstellkraft des Federmechanismus gehalten werden, welcher die Kapsel ggf. auch im Falle von vorliegenden Reibungskräften o.Ä. aus der Druckmesskapselhalterung drückt, sobald die Greifvorrichtung gelöst wird. Insbesondere ist in diesem Fall ein Federweg vorzusehen, welcher ausreichend lang ist, damit ein Nutzer die Druckmesskapsel einfach greifen kann oder, vorzugsweise, damit die Druckmesskapsel vollständig aus der Druckmesskapselhalterung ausgeworfen wird und nur noch an den daran angebrachten Leitungen hängt.

Durch die erfindungsgemäße Druckmesskapselhalterung, insbesondere durch das Zusammenspiel zwischen der Greifvorrichtung und dem Auswurfmechanismus, insbesondere dem Federmechanismus, wird zum einen eine sehr genaue, stabile Positionierung der Kapsel erreicht, ohne dass die Halterung zusätzlich eingestellt werden muss, was eine schnelle und einfache Montage der Druckmesskapsel ermöglicht, und zum anderen wird sichergestellt, dass die Druckmesskapsel schnell und einfach zu montieren und zu demontieren ist. Darüber hinaus kann dadurch, dass die Kapsel in der Halterung definiert gehalten und durch den Auswurfmechanismus gezielt ausgeworfen werden kann, ein Zustand vermieden werden, in welchem eine lockere/lecke Verbindung der Druckübertragungsleitung und der Fluidkammer vorliegt (z.B. durch eine nicht korrekt angeschlossene Luer-Lockverbindung). D.h., durch das gezielte Auswerfen gibt es nur die Zustände "konnektiert" und "nicht konnektiert". Ferner liefert insbesondere ein Einrasten der Druckmesskapsel beim Einsetzen derselben in der Halterung ein haptisches und/oder visuelles und/oder hörbares Feedback für eine korrekte Konnektierung. Verschiedene Ausführungsformen der Erfindung, bei welchen ein entsprechendes Einrasten erzielt werden kann, werden nachfolgend genauer beschrieben.

In nochmals anderen Worten ausgedrückt, hat eine Druckmesskapselhalterung, welche an einem Gehäuse einer extrakorporalen Blutbehandlungsmaschine angebracht ist oder Teil dieses Gehäuses ist, eine Vorrichtung zum formschlüssigen Halten einer Druckmesskapsel und ein oder mehrere eine entgegen der Einsetzrichtung wirkende Kraft aufbauende Elemente, z.B. Federn oder Führungskulissen. Diese/s Element/e sind derart in der Druckmesskapselhalterung bereitgestellt/montiert, um zumindest während des Lösens der Kapsel aus der Halterung eine Druckkraft (Federkraft) auf eine darin gehaltene oder eingesetzte Druckmesskapsel ausüben können, die entgegen einer Einsetzrichtung (d.h., von innen nach außen) wirkt, in welcher die Druckmesskapsel in die Halterung einsetzbar ist. Dadurch wird das Herausnehmen der Kapsel aus der Halterung unterstützt bzw. ein Auswerfen der Kapsel bewirkt. Vorzugsweise wird die Druckmesskapsel von vorne bzw. an einem Frontpanel des Gehäuses der extrakorporalen Behandlungsmaschine, gut sichtbar und einfach zugänglich in die Druckmesskapselhalterung eingesetzt. Sie kann jedoch auch seitlich, von oben oder von unten (d.h., parallel zu dem Gehäuse) eingesetzt werden.

Dadurch, dass die Druckmesskapselhalterung unmittelbar außen am Gehäuse der extrakorporalen Blutbehandlungsmaschine montiert oder integriert ist, kann ferner gewährleistet werden, dass die Fluid- oder Luftkammer der Druckmesskapsel über eine kurze, steife Druckübertragungsleitung mit einem Drucksensor verbunden ist, welcher gehäuseinnenseitig (innerhalb des Gehäuses der extrakorporalen Blutbehandlungsmaschine) bevorzugt unmittelbar hinter der Druckmesskapselhalterung vorgesehen ist und ein Drucksignal der Druckmesskapsel in ein elektrisches Messsignal wandelt. Demgemäß ist aufgrund der Anordnung der Druckmesskapsel in der Druckmesskapselhalterung eine hochgenaue, zuverlässige Druckmessung während eines Betriebs der extrakorporalen Blutbehandlungsmaschine möglich.

Der Auswurfmechanismus, insbesondere Federmechanismus, kann zum Auswerfen der Druckmesskapsel eine Druckkraft bereitstellen, indem zumindest eine Druckfeder derart in der Halterung angebracht ist, dass diese in der Einsetzrichtung hinter einer darin eingesetzten Druckmesskapsel sitzt. Alternativ ist auch eine Anordnung mit einer Zugfeder denkbar. Als Federn können für den Federmechanismus beispielsweise mechanische Federn wie Spiralfedern, Biegefedern/Blattfedern oder Tellerfedern (d.h., ein elastisch verformbares Bauteil, welches im verformten Zustand eine elastische Rückstellkraft bereitstellt) oder pneumatische Federn einzeln oder kombiniert (parallel und/oder seriell geschaltet) verwendet werden. Mögliche Ausführungen der Greifvorrichtung werden später genauer beschrieben.

Vorteilhafte Ausgestaltungen sind Gegenstand der Unteransprüche und werden nachfolgend genauer erläutert.

**In** anderen Worten ausgedrückt, ist der Federmechanismus derart ausgelegt, dass er rückseitig (in Bezug auf die Einsetzrichtung, in welcher die Druckmesskapsel in die Halterung eingesetzt wird), d.h., im Wesentlichen zwischen dem Gehäuse und der Druckmesskapsel, gegen die Druckmesskapsel drückt. Ferner drücken nicht Federn unmittelbar gegen die Druckmesskapsel, sondern ist ein Zwischenstück in Form des Druckelements bereitgestellt, wodurch eine durch den Federmechanismus erzeugte Auswurfkraft gleichmäßig und schonend auf die Druckmesskapsel übertragen werden kann. Das Druckelement kann hierfür beispielsweise ringförmig sein und an einem Außenrand der Druckmesskapsel anliegend und/oder kann eine Mulde zur Aufnahme eines Körpers der Druckmesskapsel bilden. Das Druckelement hat vorzugsweise einen metallenen (besonders druckstabilen) Stab.

Vorzugsweise ist die Greifvorrichtung über einen Schaft an dem Gehäuse montiert, wobei das Druckelement in dem Schaft aufgenommen und federnd gelagert ist. Ein Durchmesser des Schafts kann im Wesentlichen einer Ausdehnung des Greifmechanismus entsprechen, wodurch die resultierende Verbindung besonders robust ist, viel Platz zur (teilweisen) Aufnahme des Federmechanismus bzw. des Druckelements bietet und unnötige Verschneidungen und Kanten vermeidet, oder kann wesentlich schmaler als die Druckmesskapselhalterung sein, was kostengünstiger und leichter ist. Durch den Schaft kann ferner ein bestimmter Abstand zwischen dem Gehäuse und der Greifvorrichtung eingestellt sein oder werden, um ein Einsetzen der Blutleitungen zu vereinfachen. Zudem wird auf diese Weise ausreichend Platz zur rundum geschützten Aufnahme des Druckelements und dessen Federung/Lagerung bereitgestellt.

Das Anschlusselement ist komplementär zu einem Fluid-/Luftkammeranschlusselement einer Fluid- oder Luftkammer der Druckmesskapsel ausgebildet. Weist beispielsweise die Druckmesskapsel einen weiblichen Luer-Konnektor, vorzugsweise einen Luer-Slip-Konnektor, auf, so ist das Anschlusselement als männlicher Luer-(Slip-) Konnektor ausgebildet, oder umgekehrt. Alternativ kann z.B. auch eine stirnseitige Dichtfläche bereitgestellt sein, welche durch den Federmechanismus gegen eine komplementäre Fläche der Druckmesskapsel gedrückt werden kann. In diesem Fall dient der Auswurfmechanismus insbesondere zur Kraftbeaufschlagung oder zum Andrücken einer Verbindung zwischen der Kapsel und der Halterung.

Eine konische Form des Anschlusselements ermöglicht eine besonders robuste, gegenüber leichten Abweichungen unempfindliche und beim Einsetzen geführte Verbindung der Druckmesskapsel mit der Druckmesskapselhalterung. Ferner kann zwischen dem Fluid-/Luftkammeranschlusselement der Druckmesskapsel dem Anschlusselement der Druckmesskapselhalterung eine zusätzliche, manuell leicht lösbare Verbindung bestehen, welche die Druckmesskapsel auch bei einem Lösen der Greifvorrichtung hält. Insbesondere kann eine kraft- oder reibschlüssige Verbindung vorgesehen sein, beispielsweise dadurch, dass die konische Form der Anschlusselemente als Keil wirkt. Auf diese Weise wird beim Lösen der Greifvorrichtung das Druckelement zusammen mit der Druckmesskapsel durch den Auswurfmechanismus aus einer Stellung des Haltezustands herausbewegt, sodass die Greifvorrichtung mit der Druckmesskapsel nicht erneut in ein-/umgreifende Halteverbindung kommen kann. Dabei können durch die zusätzliche, manuell leicht lösbare Verbindung, insbesondere Keilverbindung, beim Lösen der Greifvorrichtung die Kapsel und die damit verbundenen Blutleitungen nicht einfach herunterfallen, sondern müssen durch einen Anwender zur Demontage zusätzlich gelöst oder abgezogen werden.

Es hat sich als zweckmäßig erwiesen, wenn an dem vorzugsweise konischen Anschlusselement des Druckelements eine Dichtung, vorzugsweise ein O-Ring, angebracht ist oder das Anschlusselement als Dichtung ausgebildet ist. Insbesondere kann die Dichtung an einer Kontaktfläche sitzen, an welcher die Anschlusselemente einander kontaktieren. Dadurch wird ein für eine besonders genaue Druckmessung benötigte Dichtigkeit zwischen den Anschlüssen der Druckmesskapsel und der Druckmesskapselhalterung erreicht. Zudem kann die Dichtung als die zusätzliche, manuell leicht lösbare Verbindung dienen und somit eine doppelte Funktion erfüllen.

Alternativ kann die Dichtung schafteingangsseitig des Druckelements derart an einer Innenfläche des Schafts angebracht sein, dass sie radial außen an dem Fluid-/Luftkammeranschlusselement der Druckmesskapsel anliegt, genauer an einer daran bereitgestellten, ggf. entsprechend nachbearbeiteten Dichtungsanlagefläche, wenn diese in die Halterung eingesetzt ist. Andererseits kann die Dichtung radial außen an der Druckmesskapsel bereitgestellt sein und kann eine Innenfläche des Schafts entsprechend als Dichtungsanlagefläche ausgebildet sein. Letzteres ist vorteilhaft hinsichtlich einer einfacheren Fertigung des Dichtungssitzes sowie der Austauschbarkeit des O-Rings, d.h., wartungsfreundlicher. In diesen Fällen verschiebt der Auswurfmechanismus bei einem Lösen der Greifvorrichtung die Dichtung in Axialrichtung relativ zu der gegenüberliegenden Dichtungsanlagefläche. Um die Dichtung zu schonen ist es von Vorteil, wenn die Dichtungsanlagefläche und/oder die Fläche, an welcher die Dichtung angebracht ist, zumindest leicht angeschrägt sind. Andererseits ist es von Vorteil, wenn diese Flächen geradezylindrisch sind, da in diesem Fall auch bei Abweichungen der Axialposition der Druckmesskapsel und der Halterung stets die gleiche Dichtwirkung gewährleistet ist. In einer derart ausgestalteten Druckmesskapselhaltevorrichtung kann ferner auf einen Druckstab verzichtet werden und kann ein alternativer Auswurfmechanismus oder Federmechanismus bereitgestellt werden, wie z.B. an einem Anlageabschnitt für eine Außenfläche der Kapsel.

Vorzugsweise ist in dem Druckelement eine Druckübertragungsleitung bereitgestellt, welche an die Druckmesskapsel anschließbar ist, um einen Druck der Druckmesskapsel an einen Drucksensor der extrakorporalen Blutbehandlungsmaschine zu übertragen. Insbesondere ist es von Vorteil, wenn eine Federkraft des Federmechanismus eingestellt ist, um das Druckelement im Haltezustand derart gegen die Druckmesskapsel zu pressen, dass eine gasdichte Verbindung zwischen der Druckmesskapsel und der Druckübertragungsleitung bereitgestellt ist. Dadurch kann die vorstehend beschriebene Dichtung des Anschlusselements des Druckelements, welche durch den Auswurfmechanismus, insbesondere Federmechanismus, gegen die Druckmesskapsel gepresst wird oder pressbar ist, eine dichte, sichere Verbindung zwischen der Fluid- oder Luftkammer der Druckmesskapsel und der Druckübertragungsleitung bereitstellen, welche die Kapsel mit einem bevorzugt im Gehäuse der extrakorporalen Blutbehandlungsmaschine angeordneten Drucksensor verbindet. Alternativ kann der Druckesensor auch im Schaft unmittelbar angeschlossen an das Druckelement bereitgestellt sein. Insbesondere werden durch die federnde Lagerung bei einem Anbringen der Druckmesskapsel auch Abweichungen in Einsetzrichtung (d.h., in der Wirkrichtung des Federmechanismus) ausgeglichen. Alternativ kann auch eine Druckübertragungsleitung in dem Schaft bereitgestellt sein, welche entsprechend an die Druckmesskapsel anschließbar ist. In diesem Fall ist das Druckelement innerhalb oder an der Druckübertragungsleitung angeordnet und ist nicht dichtend an der Kapsel angeschlossen.

Nach einem weiteren Aspekt der Erfindung ist zumindest ein Wandabschnitt bereitgestellt, durch welchen ein Aufnahmeraum zur Aufnahme der Druckmesskapsel definiert ist. Dabei kann eine zu dem Wandabschnitt im Wesentlichen senkrecht verlaufende Fläche als ein Boden des Aufnahmeraums dienen, in welchem vorzugsweise der Federmechanismus aufgenommen ist, insbesondere der Schaft angeordnet ist. Der Wandabschnitt kann zumindest derart von dem Boden des Aufnahmeraums vorstehen, dass die Druckmesskapsel darin vorzugsweise vollständig aufgenommen ist. Des Weiteren sind bevorzugt zumindest zwei einander gegenüberliegende Wandabschnitte bereitgestellt, welche zwischen sich den Aufnahmeraum definieren. Die Kapsel wird in der Halterung durch den Wandabschnitt geschützt und ist wenig anfällig für Beschädigungen durch ein Anstoßen, unbedachte Bewegungen oder Ähnliches. Vorzugsweise wird die Druckmesskapsel von einer Vorderseite, d.h., zu dem Boden hin und parallel zu den Wandabschnitten, in die Druckmesskapselhalterung eingesetzt. Grundsätzlich ist es jedoch auch möglich, die Kapsel seitlich oder von oben, insbesondere zwischen zwei oder mehr Wandabschnitten, in die Halterung einzuschieben. Ggf. kann in diesem Fall eine Führung für die Kapsel an den Wandabschnitten vorgesehen sein.

Vorteilhafter Weise ist der zumindest eine Wandabschnitt mit einem Schlitz zur Aufnahme zumindest eines Blutkammeranschlusses der Druckmesskapsel versehen. Der Schlitz dient demgemäß dazu, die Kapsel hinsichtlich ihrer Blutkammeranschlüsse lagefestzulegen bzw. eine Lage ihrer Blutkammeranschlüsse festzulegen, und ist im Wesentlichen U-förmig und/oder dem Durchmesser des Blutkammeranschlusses derart angepasst, dass er diesen während des Einsetzens der Druckmesskapsel führt und im eingesetzten Zustand in drei Richtungen (d.h., in Richtung eines Schlitzendes sowie beidseitig quer dazu) formschlüssig hält. Das heißt, der Schlitz kann zur Aufnahme und Führung des Kapselabschnitts dienen. Dadurch kann ein Einsetzen in einer falschen Ausrichtung nahezu vermieden werden. Durch den Schlitz kann ferner erreicht werden, dass die Druckmesskapsel auch während einer Behandlung in den drei Richtungen lagefestgelegt ist. Wenn der Schlitz hauptsächlich in Einsetzrichtung der Kapsel in die Halterung verläuft, wird dies auch als Axialschlitz bezeichnet. Verläuft beispielsweise der (Axial-)Schlitz senkrecht zu einem Boden des Aufnahmeraums, insbesondere hin zu einem Gehäuse der extrakorporalen Blutbehandlungsmaschine oder parallel zu dem Schaft, kann erreicht werden, dass ein Verdrehen der Druckmesskapsel während einer extrakorporalen Blutbehandlung vermieden werden kann.

Ferner wird bevorzugt, wenn eine Innenumfangsfläche des zumindest einen Wandabschnitts ausgebildet ist, um als Führung für die Druckmesskapsel zu dienen. Z.B. kann die Kapsel in Einsetzrichtung im Wesentlichen rund sein und kann die Innenumfangsfläche einen entsprechenden Durchmesser oder eine entsprechende Krümmung aufweisen. Dies, ebenso wie das Bereitstellen des Schlitzes, kann ein Verkippen der Kapsel und ein dadurch ggf. bedingtes fehlerhaftes Anschließen vermeiden.

Nach einer vorteilhaften Ausführungsform bildet die Greifvorrichtung zumindest einen Schnapphaken aus, welcher dazu ausgebildet ist, bei einem Einsetzen der Druckmesskapsel hinter die Druckmesskapsel zu greifen. D.h., zumindest ein Haken ist bereitgestellt, welcher sich an der Druckmesskapselhalterung in der Einsetzrichtung der Druckmesskapsel, insbesondere parallel zu dem vorstehend genannten zumindest einen Wandabschnitt, erstreckt und an seinem freien Ende eine zum Druckmesskapselhalterungsinneren (d.h., in den Aufnahmeraum hinein) vorstehende Nase aufweist. Der/die Schnapphaken können biegefederartig nach außen aufgebogen werden, insbesondere dadurch, dass die Kapsel beim Einsetzen in die Halterung die Nase kontaktiert und diese nach außen drückt. Ein Einsetzen der Druckmesskapsel in die Halterung durch einen Nutzer erfolgt dadurch besonders einfach und schnell. Der/die Schnapphaken können ferner einen (zusätzlichen) Wandabschnitt bilden, welcher zusätzlich oder alternativ zu dem/den vorstehend beschriebenen Wandabschnitt/en den Aufnahmeraum bildet.

Beispielsweise kann ein einzelner Schnapphaken bereitgestellt sein, was dahingehend vorteilhaft ist, dass die Verbindung einhändig lösbar ist. D.h., ein Nutzer muss zum Lösen der Greifvorrichtung von der Druckmesskapsel lediglich den einzelnen Schnapphaken mit einer Hand nach außen biegen, woraufhin der Auswurfmechanismus, insbesondere der Federmechanismus, die Kapsel derart aus der Greifvorrichtung heraus drückt, dass ein erneutes in Eingriff kommen beim Loslassen des Schnapphakens nicht möglich ist. In diesem Fall kann ein erster, den Aufnahmeraum definierender und diesen vorzugsweise mindestens halb, weiter vorzugsweise zu mehr als zwei Dritteln umgebender Wandabschnitt bereitgestellt sein, welche insbesondere die zwei senkrecht zum Boden des Aufnahmeraums verlaufenden Schlitze zur Aufnahme der Blutkammeranschlüsse aufweist. Der Schnapphaken kann in diesem Fall in einer Lücke des ersten Wandabschnitts angeordnet sein, um einen zweiten Wandabschnitt zu bilden. Alternativ können zwei einander gegenüberliegende Schnapphaken bereitgestellt sein, welche entsprechend beidhändig zu lösen sind. Zwischen den Schnapphaken, insbesondere winkelversetzt (vorzugsweise um 90°) dazu, können zwei Wandabschnitte, insbesondere mit Schlitzen, ausgebildet sein.

Die Druckmesskapselhalterung nach dieser Ausführungsform kann beispielsweise spritzgegossen werden, wobei der zumindest eine Wandabschnitt, der zumindest eine Schnapphaken, ein Boden des Aufnahmeraums sowie gegebenenfalls der Schaft stoffeinstückig gefertigt sind. Dies ermöglicht eine besonders kostengünstige Herstellung der Druckmesskapselhalterung.

In anderen Worten ausgedrückt, ist die Halterung mit einem oder zwei (Plastik-) Haken ausgestattet, welche ausgelegt sind, um die Druckmesskapsel (POD) zu blockieren und in Position zu halten. Ferner sind die (Plastik-) Haken dazu ausgelegt, die Druckmesskapsel einfach zu lösen und um die Kapsel mithilfe von einem oder zwei Fingern, einem für jeden Haken, freizugeben.

Nach einer anderen vorteilhaften Ausführungsform ist der zumindest eine Schlitz ein quer zur Einsetzrichtung, insbesondere in Umfangsrichtung um den Aufnahmeraum, verlaufender Umfangsschlitz, durch welchen außenseitig (einem Boden des Aufnahmeraums entgegengesetzt) die Greifvorrichtung als zumindest eine vorzugsweise elastisch aufbiegbare, in Umfangsrichtung verlaufende Klammer abgeteilt oder ausgebildet ist, wobei der zumindest eine Blutkammeranschluss durch ein Drehen der Druckmesskapsel in den Umfangsschlitz einsetzbar ist bzw. unter die Klammer schiebbar ist.

Das heißt, die Druckmesskapselhalterung nach dieser Ausführungsform weist eine vorzugsweise umlaufende Wand auf, welche zusammen mit dem Boden den Aufnahmeraum definiert. Die Wand weist zumindest einen Schlitz auf, welcher in der Wand parallel zu dem Boden, in Umfangsrichtung verläuft. Der zumindest eine Schlitz öffnet sich hin zu einem Rand der Wand. Um dies zu ermöglichen ist der zumindest eine Schlitz an seinem Eingang in Axialrichtung (d.h., senkrecht oder geneigt weg vom Boden) abgeknickt und/oder hat die Wand in jedem Bereich, in welchem der/die Schlitz/e verlaufen, einen Vorsprung, in welchem sich der jeweilige Schlitz seitlich/in Umfangsrichtung öffnet. Ähnlich wie bei der vorstehend beschriebenen Ausführungsform kann die Druckmesskapselhalterung nach dieser Ausführungsform besonders einfach und kostengünstig, insbesondere stoffeinstückig, vorzugsweise durch Spritzgießen, hergestellt werden.

In anderen Worten ausgedrückt, weist die Halterung zwei gekrümmte Plastikhaken (Klammern) auf, welche dazu ausgelegt sind, die Druckmesskapsel durch Einhängen der Schlauchanschlüsse/Blutkammeranschlüsse in Position zu halten, wobei bei der Halterung nach dieser Ausführungsform die Verbindung der Druckmesskapsel (POD) durch eine partielle Rotation der Komponente (Kapsel) erreicht wird. Gleichermaßen erfolgt das Lösen/Herausnehmen der Druckmesskapsel durch eine partielle Rotation der Kapsel in umgekehrter Richtung.

Um die Druckmesskapsel in die Druckmesskapselhalterung einzusetzen wird in dieser Ausführungsform die Kapsel derart positioniert, dass deren Blutkammeranschluss oder Blutkammeranschlüsse im Eingang des Umfangsschlitzes liegt oder liegen. Anschließend wird die Druckmesskapsel derart (partiell) gedreht, dass der Blutkammeranschluss sich durch den Umfangsschlitz bewegt und an dessen Ende in einer Endlage stoppt/anschlägt. D.h., ein Einsetzen und Entnehmen der Kapsel in die/aus der Halterung erfolgt bajonettartig und ist für den Nutzer besonders schnell, einfach und einhändig durchführbar.

Insbesondere ist es von Vorteil, wenn der zumindest eine Umfangsschlitzes an seinem (Sack-) Ende verbreitert ist, um eine verrastbare Anschlussaufnahme bereitzustellen, welche die Endlage des jeweiligen Blutkammeranschlusses definiert. Vorzugsweise entsprechen die Abmessungen (der Durchmesser) der Anschlussaufnahme den Abmessungen des Blutkammeranschlusses. In diesem Fall sind durch die Umfangswand gebildete Klammern/Flügel, welche außenseitig (entgegen der Einsetzrichtung gewandt oder dem Boden des Aufnahmeraums entgegengesetzt) des Umfangsschlitzes stehen bleiben oder durch den Umfangsschlitz teilweise von der Umfangswand abgetrennt sind, elastisch aufbiegbar, sodass die Kapsel mit ihren Blutkammeranschlüsse in einer Endlage am Ende des Schlitzes verrastet. Hierfür ist es insbesondere vorteilhaft, wenn an einem äußeren/stirnseitigen Rand der Wand neben dem Ende des Umfangsendes oder der Anschlussaufnahme, also hinter einem Ansatz der der Klammer/des Flügels ein Rücksprung vorgesehen ist. Durch einen solchen Rücksprung wird die Flexibilität oder die elastische Aufweitbarkeit der außenseitig des Umfangsschlitzes bereitgestellten Flügel/Klammern erhöht wodurch beispielsweise der Nutzer weniger Kraft benötigt, um die Druckmesskapsel zu verrasten und aus der Verrastung zu lösen.

Als Auswurfmechanismus kann der Federmechanismus vorgesehen sein, wobei der Umfangsschlitz bevorzugt zumindest an seinem inneren, dem Eingang entgegengesetzten Ende (ggf. ausschließlich) in Umfangsrichtung verläuft. Alternativ oder zusätzlich kann der Umfangsschlitz leicht schräg verlaufen (d.h., sich um den Aufnahmeraum und schräg hin zum Boden erstrecken), sodass der Umfangsschlitz eine Führungskulisse als den Auswurfmechanismus bildet, durch deren schrägen Verlauf die Kapsel beim Herausnehmen aus der Halterung auch senkrecht zu dem Boden aus der Halterung herausbewegt wird. Ferner kann durch die so gebildete Führungskulisse beim Einsetzen der Kapsel eine Anpresskraft in Richtung des Bodens und einer daran bereitgestellten Anschlussstelle oder Dichtung erreicht werden.

Nach noch einer weiteren bevorzugten Ausführungsform weist die Greifvorrichtung einen an einer Außenseite des zumindest einen Wandabschnitts drehbar gelagerten, hülsenförmigen Drehriegel oder Riegelring auf, der zumindest einen L-förmigen Schlitz aufweist. Der L-förmige Schlitz hat einen Axialabschnitt, welcher sich entgegen einer Einsetzrichtung an einem (stirnseitigen) Außenrand des Drehriegels öffnet. Der Axialabschnitt überlagert in einer Offenstellung des Drehriegels den zumindest einen in dem Wandabschnitt bereitgestellten, axial verlaufenden Schlitz (Axialschlitz) derart, dass der zumindest eine Blutkammeranschluss in den jeweiligen L-förmigen Schlitz und axialen Schlitz einsetzbar ist. Des Weiteren hat der L-förmige Schlitz einen Umfangsabschnitt, welcher ein in Umfangrichtung verlaufendes Schlitzende ausbildet und dazu angepasst ist, den zumindest einen Blutkammeranschluss in einer durch Verdrehen des Drehriegels erreichbaren Schließstellung zusammen mit dem Axialschlitz lagefestgelegt zu halten. Die Druckmesskapselhalterung bzw. die Greifvorrichtung nach dieser Ausführungsform hält die Druckmesskapsel besonders stabil und sicher. Ferner ist es von Vorteil, dass der Drehriegel als umlaufende Schutzwand zum Schutz der Druckmesskapsel dient. Optional kann der Drehriegel drehfedergelagert sein, um in die Schließstellung vorgespannt zu sein.

Ähnlich wie bei der vorstehend beschriebenen Ausführungsform mit dem Umfangsschlitz kann der Auswurfmechanismus durch den Federmechanismus und/oder durch eine Führungskulisse gebildet werden, wobei die Führungskulisse in diesem Fall durch den Umfangsabschnitt des L-förmigen Schlitzes bereitgestellt ist. Ist nur der Federmechanismus bereitgestellt, verläuft der Umfangsabschnitt vorzugsweise ausschließlich in Umfangsrichtung. Die Führungskulisse wird dadurch ausgebildet, dass der Verlauf des Umfangsabschnitts des L-förmigen Schlitzes zusätzlich zu seiner Umfangskomponente eine Axialkomponente hat (rund um den Aufnahmeraum sowie schräg in Richtung des Bodens verläuft). Dadurch drückt beim Verdrehen/Schließen des Drehriegels ein äußerer Rand des L-förmigen Schlitzes gegen den jeweiligen Blutkammeranschluss, um die Kapsel in den Aufnahmeraum hinein / zum Boden hin zu drücken und gegebenenfalls zwischen dem Fluid-/Luftkammeranschlusselement der Kapsel und dem Anschlusselement der Halterung (z.B. dem Schaft oder dem Druckelement) vorhandene Dichtungen aneinander zu pressen. Ist ein federgelagertes Druckelement vorhanden, d.h., hat der Auswurfmechanismus sowohl einen Federmechanismus als auch eine Führungskulisse, wird beim Lösen der Kapsel der Federmechanismus zum Auswerfen der Kapsel durch einen unteren Rand des L förmigen Schlitzes unterstützt, was insbesondere aufgrund der erreichbaren, hohen Anpresskraft und dadurch möglicherweise entstehenden Verklemmungen vorteilhaft ist, da durch die Führungskulisse das Auswerfen weniger Kraft kostet und zudem kontrollierter erfolgt. Das heißt, bei einer Entfernung der Kapsel wird ein automatisches Herausdrücken der Kapsel bereitgestellt.

In anderen Worten ausgedrückt, ist die Druckmesskapselhalterung mit einem rotierenden Außenring ausgestattet, welcher dazu ausgelegt ist, während des Einsetzens der Druckmesskapsel zu rotieren bzw. manuell rotiert zu werden. Ferner kann der rotierende Außenring dazu ausgelegt sein, die Kapsel während der Rotation (aus der Halterung) herauszudrücken.

Nach noch einer weiteren vorteilhaften Ausführungsform bildet die Greifvorrichtung eine über ein scharnierartiges Gelenk angebrachte oder verschiebbar gelagerte Riegellasche derart aus, dass diese sich in einer Schließstellung an einer dem Federmechanismus gegenüberliegenden Seite über die Druckmesskapsel erstreckt, um diese entgegen einer Federkraft des Federmechanismus in der Halterung zu halten.

Vorzugsweise bildet die Riegellasche einem dem Gelenk gegenüberliegenden Ende eine Rastkante oder einen Rasthaken/Rastvorsprung, um in der Schließstellung mit einem zugehörigen, starr an der Halterung vorgesehenen Haken oder Kante oder Rille zu verrasten. Vorzugsweise ist der Haken oder der Vorsprung ein Schnapphaken oder eine Rastnase und erfolgt das Verrasten durch ein Einschnappen derselben. Alternativ kann ein manuell bewegbarer Haken, Bügel oder Stift (drehbar oder verschiebbar gelagert) vorgesehen sein, um einen hinterschneidenden Halteeingriff zwischen der Riegellasche und einem Hauptkörper der Druckmesskapselhalterung herzustellen. Ferner ist es von Vorteil, wenn die Riegellasche fachwerkartig ausgebildet ist, sodass die Druckmesskapsel, insbesondere ein die Luft-und Blutkammer aufweisender Hauptkörper, auch bei einer geschlossenen Riegellasche sichtbar ist.

Insbesondere wenn die Riegellasche über das scharnierartige Gelenk angebracht ist, kann ferner die Riegellasche als eine Biegefeder ausgelegt sein, welche eine zusätzliche Anpresskraft auf die in die Halterung eingelegte Druckmesskapsel ausüben kann. Alternativ ist denkbar, die Riegellasche als eine starr an der Druckmesskapselhalterung angebrachte Biegefeder auszulegen, welche zum Einsetzen der Druckmesskapsel aufgebogen wird und welche die Kapsel anschließend (ausschließlich) durch ihre Federkraft in der Halterung hält.

In anderen Worten ausgedrückt, ist die Halterung mit einem Plastikdeckel ausgestattet, welcher dazu ausgelegt ist, die Kapsel in der angemessenen Position zu halten, wobei der Plastikdeckel in der geschlossenen Position durch einen Haken gehalten wird. Die Kapsel wird durch den Deckel blockiert. Die Halterung ist insbesondere mit einem Luer-Lock- Konnektor ausgestattet.

In jeder der vorstehend beschriebenen Ausführungsformen ist es von Vorteil, wenn die Druckmesskapselhalterung derart ausgelegt ist, dass der Hauptteil/Hauptkörper der Druckmesskapsel mit den durch die Membran getrennten Blut- und Fluid-/Luftkammern sichtbar ist, sodass ein Nutzer eine Positionierung/Einstellung der Membran, welche die beiden Kammern voneinander trennt, einfach überwachen kann.

Zusammenfassend wird die der Erfindung zugrunde liegende Aufgabe gelöst durch eine direkte Verbindung der Kapsel (POD) mit der extrakorporalen Blutbehandlungsmaschine oder Dialysemaschine (deren Gehäuse), in welcher die Druckmesskapsel während einer extrakorporalen Blutbehandlung (Dialysebehandlung) in der korrekten Position gehalten wird. Die Halterung zielt darauf ab, die Kapsel an einem Frontpanel der extrakorporalen Blutbehandlungsmaschine einzuhalten/zu befestigen. Ferner erlaubt die Halterung eine Inspektion der Einstellung der Kapselmembran (die die Kammern der Kapsel voneinander trennende Membran). Die Kapsel wird durch die Halterung in der korrekten Position gehalten und eine Rotation der Kapsel wird vermieden. Die Halterung erlaubt eine Übertragung eines Drucks zwischen den Blutleitungen und der extrakorporalen Blutbehandlungsmaschine. Ein Abknicken der Druckmesskapsel bezüglich Schläuchen (z.B. einer Druckübertragungs(schlauch)leitung zwischen der Fluid-/Luftkammer der Kapsel und dem Drucksensor) wird vermieden. Die Halterung erlaubt ein Verbinden von Plastikschläuchen. Zudem ist die Halterung dazu ausgelegt, um einfach reinigbar zu sein und von der Maschine zu Wartungszwecken einfach abnehmbar zu sein. Darüber hinaus kann die Halterung mit einem Verbinder aus Weichplastik (Anschlusselement und/oder Dichtung) ausgestattet sein, um die Kapsel anzuschließen, wobei der Verbinder eine Dichtung mit der Kapsel garantiert. Der Verbinder aus Weichplastik ist auf einem metallenen Schaft (Druckelement) montiert. Ferner ist die Halterung dazu ausgelegt, die Kapsel bei deren Entfernung/Abnahme (automatisch) auszuwerfen.

### Figurenbeschreibung

Nachfolgend wird die vorliegende Erfindung anhand bevorzugter Ausführungsformen beschrieben. Diese sind jedoch nur veranschaulichender Natur und sollen den Schutzumfang der vorliegenden Erfindung nicht einschränken. Ferner werden bei der Beschreibung der verschiedenen Ausführungsformen für gleiche Bauteile dieselben Bezugszeichen verwendet, um redundante Beschreibungen derselben zu vermeiden.
Fig. 1 zeigt eine erfindungsgemäße Druckmesskapselhalterung und eine Druckmesskapsel während eines Einsetzvorgangs nach einer ersten Ausführungsform.
Fig. 2 zeigt die Druckmesskapselhalterung nach der ersten Ausführungsform und die darin gehaltenen Druckmesskapsel.
Fig. 3 zeigt eine erfindungsgemäße Druckmesskapselhalterung nach einer Modifikation der ersten Ausführungsform.
Fig. 4 zeigt die Druckmesskapselhalterung nach der Modifikation der ersten Ausführungsform und die darin gehaltenen Druckmesskapsel.
Fig. 5 zeigt eine erfindungsgemäße Druckmesskapselhalterung nach einer zweiten Ausführungsform.
Fig. 6 zeigt die Druckmesskapselhalterung nach der zweiten Ausführungsform und die darin eingesetzte Druckmesskapsel.
Fig. 7 zeigt eine erfindungsgemäße Druckmesskapselhalterung nach einer dritten Ausführungsform.
Fig. 8 zeigt die Druckmesskapselhalterung nach der dritten Ausführungsform und die darin eingesetzte Druckmesskapsel.
Fig. 9 zeigt eine erfindungsgemäße Druckmesskapselhalterung nach einer vierten Ausführungsform.
Fig. 10 zeigt die Druckmesskapselhalterung nach der vierten Ausführungsform und die darin gehaltene Druckmesskapsel.
Fig. 11 zeigt einen Federmechanismus am Beispiel der ersten Ausführungsform.
Fig. 12, Fig. 13 und Fig. 14 zeigen vorteilhafte Varianten zur Anbringung einer Dichtung am Beispiel der ersten Ausführungsform.

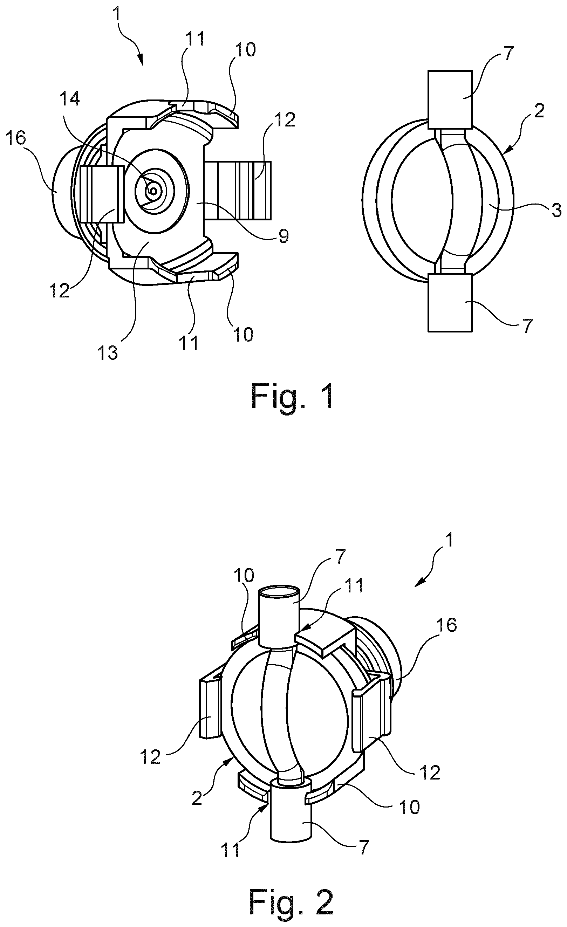

Fig. 1 zeigt eine erste Ausführungsform der erfindungsgemäßen Druckmesskapselhalterung 1, nachfolgend auch kurz als Halterung 1 bezeichnet, während eine Druckmesskapsel 2, nachfolgend auch kurz als Kapsel 2 bezeichnet, frontal darin eingesetzt wird (während eines Einsetzvorgangs). Die Druckmesskapsel 2 weist einen Hauptkörper 3 auf, welcher in diesem Beispiel im Wesentlichen rund ist und eine starre Kapsel bildet, deren Innenraum durch eine Membran 4 zur Druckübertragung in zwei Kammern, eine Blutkammer 5 und eine Luftkammer 6, geteilt ist. An einer in dieser Ansicht dargestellten Vorderseite der Druckmesskapsel 2 sind Blutkammeranschlüsse 7 vorgesehen, welche dazu angepasst sind, die Druckmesskapsel 2, genauer deren Blutkammer 5, mit einer Blutschlauchleitung einer extrakorporalen Blutbehandlungsmaschine fluidleitend zu verbinden. An der Rückseite und hier nicht dargestellt weist die Druckmesskapsel 2 einen mit der Luftkammer 6 fluidleitend verbundenen, vorzugsweise konischen Luftkammeranschluss 8, insbesondere einen Luer-Konnektor auf.

Bei der nachfolgenden Beschreibung der Ausführungsformen ist zu beachten, dass die hier beispielhaft beschriebene, in sämtlichen Ausführungsformen vorausgesetzte Druckmesskapsel 2 frontal in die nach vorne hin offene Halterung 1 eingesetzt wird. Da die Kapsel 2 ferner rund ist und die zugehörige Halterung zumindest eine entsprechend runde Aufnahme bildet, wird nachfolgend eine Einsetzrichtung auch als Axialrichtung bezeichnet und bezieht eine Umfangsrichtung sich auf diese runde Form. Es ist jedoch verständlich, dass die Druckmesskapsel anders, z.B. quaderförmig, ausgebildet sein kann oder seitlich in die Halterung einsetzbar sein kann, ohne das Funktionsprinzip der vorliegenden Erfindung zu ändern.

Die Druckmesskapselhalterung 1 bildet einen topfartigen Aufnahmeraum 9, welcher dazu angepasst ist, die Druckmesskapsel 2 aufzunehmen. Der Aufnahmeraum 9 weist an zwei einander gegenüberliegenden Seiten Wände oder Wandabschnitte 10 auf, die einen Teil einer Hülse bilden, deren Innenumfang im Wesentlichen einem Außenumfang der Druckmesskapsel 2 entspricht/entsprechen, um diese aufzunehmen und zu führen. Die Wandabschnitte 10 weisen zwei einander diametral gegenüberliegende, in Einsetzrichtung verlaufende Schlitze 11 (nachfolgend als Axialschlitze bezeichnet) auf, welche dazu ausgebildet sind, die Blutkammeranschlüsse 7 der Druckmesskapsel 2 aufzunehmen, und dadurch eine drehfeste Lage der Druckmesskapsel 2 relativ zu der Druckmesskapselhalterung 1 zu gewährleisten. Darüber hinaus weist der Aufnahmeraum 9 zwei einander gegenüberliegende, zu den Wandabschnitten 10 und den darin vorgesehenen Axialschlitzen 11 winkelversetzte (vorzugsweise um 90°) Schnapphaken oder Federhaken 12 auf, welche beim Einsetzen der Druckmesskapsel 2 elastisch nach radial außen gebogen werden und, wenn die Druckmesskapsel 2 ihre Endlage erreicht hat, zurückfedern oder einschnappen und dabei mit Hakenvorsprüngen, welche sich radial nach innen erstrecken, um einen Rand der Druckmesskapsel 2 greifen. D.h., die Federhaken 12 dienen als eine Greifvorrichtung.

Der durch die Halterung 1 gebildete topfartige Aufnahmeraum 9 hat ferner einen Boden 13, in welchem ein zu dem Luftkammeranschluss 8 der Druckmesskapsel 2 (Anschlussabschnitt der Kapsel) kompatibler Halterungsanschluss 14 (Halterungsanschlusselement / Anschlussabschnitt der Halterung), insbesondere ein passender Luer-Konnektor, bereitgestellt ist. Der Halterungsanschluss 14 ist in der Halterung 1, insbesondere in einer Öffnung im Boden 13 der Halterung 1, federgelagert montiert, wobei ein zugehöriger Federmechanismus 15, welcher später genauer beschrieben wird, in einem Schaft 16 der Halterung 1 untergebracht ist.

Wird die Druckmesskapsel 2 in die Halterung 1 eingesetzt, werden der Halterungsanschluss 14 und der Luftkammeranschluss 8 miteinander verbunden und durch den Federmechanismus 15 dichtend aneinander gepresst. Ferner dient der Federmechanismus 15 dazu, beim Lösen der Greifvorrichtung bzw. der Federhaken 12 die Druckmesskapsel 2 aus dem Aufnahmeraum 9 herauszudrücken oder auszuwerfen, dient also als ein Auswurfmechanismus. Ein Federweg und eine Federkraft des Federmechanismus 15 sind entsprechend dimensioniert. Der Schaft 16 der Halterung 1 dient ferner dazu, die Halterung 1 an einem Gehäuse einer extrakorporalen Blutbehandlungsmaschine zu befestigen. Genauer ausgedrückt, weist Schaft 16 einen Kragen zur Anlage an dem Gehäuse der extrakorporalen Blutbehandlungsmaschine und somit zur Definition einer Lage der Halterung 1 relativ dazu auf.

Fig. 2 zeigt die erste Ausführungsform der erfindungsgemäßen Druckmesskapselhalterung 1, in welche die Druckmesskapsel 2 eingesetzt ist. Es ist gut erkennbar, dass die Blutkammeranschlüsse 7 der Druckmesskapsel 2 in den Axialschlitzen 11 der Halterung 1 liegen und die Druckmesskapsel 2 auf diese Weise gegen Verdrehung gesichert ist. Ferner ist ersichtlich, dass die Federhaken 12 um einen Rand der Druckmesskapsel 2 greifen und diese somit in Axialrichtung/Einsetzrichtung lagefixieren.

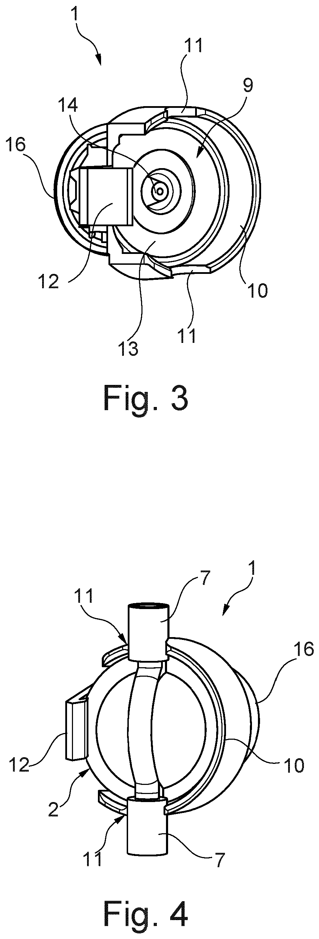

Fig. 3 und Fig. 4 zeigen eine Druckmesskapselhalterung 1 nach einer Modifikation der ersten Ausführungsform der Erfindung einzeln sowie mit einer eingesetzten Druckmesskapsel 2. Diese Ausführungsform entspricht abgesehen von der Modifikation im Wesentlichen der ersten Ausführungsform, weshalb nachfolgend nur Unterschiede erläutert werden. Im Unterschied zu der ersten Ausführungsform weist die hier dargestellte Druckmesskapselhalterung 1 nur einen einzelnen Federhaken 12 als eine Greifvorrichtung auf. Des Weiteren ist nur ein Wandabschnitt 10 vorgesehen, welcher sich hülsenartig rund um den Aufnahmeraum 9 erstreckt und lediglich im Bereich des Federhakens 12 unterbrochen ist. Das heißt, an der Stelle, an welcher in der Halterung 1 gemäß der ersten Ausführungsform der zweite Federhaken 12 wäre, ist ein einzelner, durchgehender Wandabschnitt 10 vorgesehen.

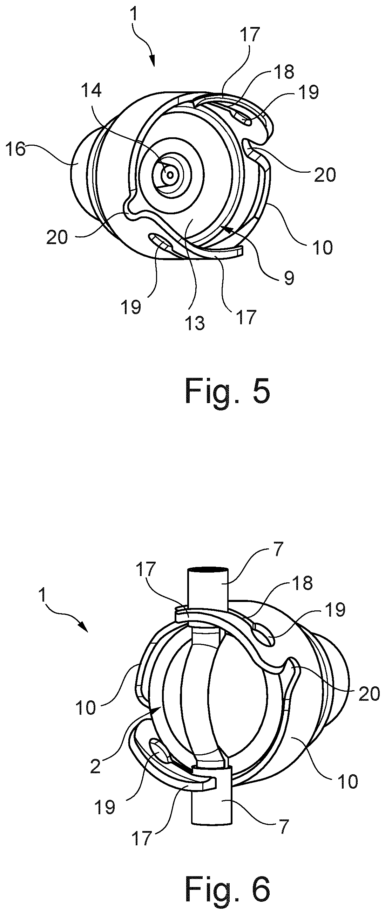

Fig. 5 zeigt eine zweite Ausführungsform der vorliegenden Erfindung. Diese entspricht weitgehend der vorstehenden ersten Ausführungsform und unterscheidet sich davon im Wesentlichen anhand der Greifvorrichtung sowie anhand einer zugehörigen Einsetzweise, welche nachfolgend erläutert werden. Der von der Halterung 1 gebildete Aufnahmeraum 9 ist von einer in Einsetzrichtung/Axialrichtung undurchbrochenen, umlaufenden, hülsenartigen Wand 10 umgeben. An zwei einander diametral gegenüberliegenden Seiten bildet die Wand 10 axiale Fortsätze 17 aus. In jedem dieser Fortsätze 17 ist ein in einer gleichen Umfangsrichtung verlaufender (d.h., drehsymmetrisch), sich teilweise um den Aufnahmeraum 9 erstreckender Schlitz oder Umfangsschlitz 18 eingebracht, welcher zur Aufnahme der Blutkammeranschlüsse 7 der Druckmesskapsel 2 dient. D.h., die Fortsätze 17 sind durch die Umfangsschlitze 18 derart von der Wand 10 abteilt oder teilweise abgetrennt, dass sie stirnseitig (an einer vorderen Kante) von der Wand 10 erstreckende, in Umfangsrichtung verlaufende Klammern oder Flügel als die Greifvorrichtung bilden.

Die Umfangsschlitze 18 sind jeweils in ihrem Verlauf gleichmäßig schmal, wobei sie sich gegebenenfalls an ihren Eingängen geringfügig weiten und nach vorne hin / stirnseitig öffnen. In anderen Worten ausgedrückt, kann der Umfangschlitz 18 sich über den zugehörigen Fortsatz 17 hinaus in einen zwischenliegenden Abschnitt der Wand 10 erstrecken, um an einem Übergang zwischen dem Fortsatz 17 und diesem zwischenliegenden Abschnitt der Wand 10 eine Art Aufnahmetrichter auszubilden. Ferner bilden die Umfangsschlitze jeweils an ihrem anderen, geschlossenen (Sack-) Ende eine rund aufgeweitete Anschlussaufnahme 19 oder eine Verbreiterung zur Verrastung der Blutkammeranschlüsse 7 aus. Hinter den Fortsätzen 17, an der Seite der Anschlussaufnahmen 19, weist die Wand 10 einen Rücksprung 20 auf, wodurch eine Biegsamkeit der durch die Fortsätze 17 gebildeten Klammern/Flügel erhöht ist.

Um die Druckmesskapsel 2 in die Halterung 1 einzusetzen, wird die Druckmesskapsel 2, wie in Fig. 6 gezeigt, derart in/an den Aufnahmeraum 9 gesetzt, dass die Blutkammeranschlüsse 7 an den einander diametral gegenüberliegenden Eingängen der Umfangsschlitze 18 bzw. den dadurch gebildeten Aufnahmetrichtern liegen. Wird nun die Druckmesskapsel 2 in Umfangsrichtung relativ zu der Druckmesskapselhalterung 1 bajonettähnlich verdreht, gleiten die Blutkammeranschlüsse 7 durch die Umfangsschlitze 18, wobei die durch die Fortsätze 17 gebildeten, in Umfangsrichtung verlaufenden Klammern elastisch in Axialrichtung aufgeweitet werden und sich ggf. geringfügig verdrehen. Erreichen die Blutkammeranschlüsse 7 die rund aufgeweiteten Anschlussaufnahmen 19 der Umfangsschlitze 18, stellen sich die elastisch verformten Klammern zurück und rasten somit die Blutkammeranschlüsse 7 in einer durch die Anschlussaufnahmen 19 gebildeten Endposition ein. Bei diesem Einsetzvorgang sowie bei einem genau umgekehrt verlaufenden Lösen der Kapsel aus der Halterung 1 dienen die Umfangsschlitze 18 (genauer, die Umfangsschlitze 18 einfassenden Wandränder der Klammer und eines hinteren Wandabschnitts) ggf. als eine Führungskulisse, um zumindest einen Teil des erfindungsgemäßen Auswurfmechanismus zu bilden. Zusätzlich oder alternativ ist am/im Boden 13 der Halterung ein Federmechanismus 15, wie er später genauer beschrieben wird, als zumindest ein Teil des Auswurfmechanismus bereitgestellt. Der Federmechanismus 15 und die Führungskulisse können auch einen kombinierten Auswurfmechanismus bilden.

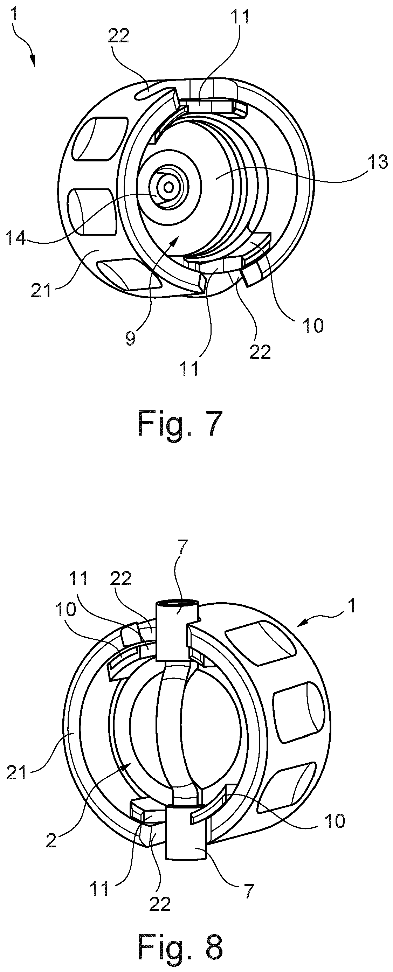

Fig. 7 und Fig. 8 zeigen eine dritte Ausführungsform der vorliegenden Erfindung. Wie auch die vorstehend beschriebenen Ausführungsformen weist die hier dargestellte Halterung 1 einen topfartigen Aufnahmeraum 9 auf. Dieser Aufnahmeraum 9 wird von einer Innenhülse mit einem Boden 13 und einer Wand mit zwei einander diametral gegenüberliegenden, sich in Einsetzrichtung/Axialrichtung erstreckenden Wandabschnitten 10 gebildet. In den Wandabschnitten 10 sind, ähnlich wie in Fig. 1, (jeweils) ein Axialschlitz 11 zur Aufnahme der Blutkammeranschlüsse 7 der Druckmesskapsel 2 vorgesehen. Eine Innenfläche der Wandabschnitte 10 dient zur Aufnahme und Führung eines Außenumfangs der Druckmesskapsel 2. Eine Außenfläche der Wandabschnitte 10 dient andererseits dazu, einen Riegelring 21 relativ zu dem Aufnahmeraum 9 drehbar zu halten und zu lagern. An einer Außenumfangsfläche hat der Riegelring 21 in Umfangsrichtung verteilte Mulden, welche zur besseren Greifbarkeit durch einen Anwender dienen. Der Riegelring 21 ist eine Hülse, welche zwei einander diametral gegenüberliegende, im wesentlichen L-förmige Schlitze 22 aufweist, die in der gleichen Richtung wie die Axialschlitze 11, zum stirnseitigen/vorderen Rand des Riegelrings 21 hin, geöffnet sind und dort einen Axialabschnitt bilden. Ferner weist jeder L-förmige Schlitz 22 einen Umfangsabschnitt auf, welcher von dem Axialabschnitt in einem Winkel von gleich oder größer 90° abknickt und sich somit zumindest teilweise in Umfangsrichtung um den Aufnahmeraum 9 erstreckt.

Um die Druckmesskapsel 2 in die Halterung 1 der dritten Ausführungsform zu befestigen, wird der Riegelring 21 relativ zu dem Aufnahmeraum 9 derart gedreht, dass die Öffnungen/Eingänge der Axialschlitze 11 und die Öffnungen/Eingänge der Axialabschnitte der L-förmigen Schlitze 22, wie in Fig. 8 gezeigt, miteinander fluchten. Anschließend wird die Druckmesskapsel 2 derart in den Aufnahmeraum 9 eingesetzt, dass die Blutkammeranschlüsse 7 in den Axialschlitzen 11 und den Axialabschnitten der L-förmigen Schlitzen 22 liegen. Wird nun der Riegelring 21 relativ zu der Druckmesskapsel 2 verdreht, schieben sich die Umfangsabschnitte der L-förmigen Schlitze 22 über die Blutkammeranschlüsse 7. Auf diese Weise wird die Druckmesskapsel 2 durch die Axialschlitze 11 gegen Verdrehung gesichert und durch den Umfangsabschnitt der L-förmigen Schlitze 22 in Axialrichtung gesichert.

Ähnlich wie in der vorstehend beschriebenen zweiten Ausführungsform kann ein Auswurfmechanismus durch einen im/am Boden 13 der Halterung bereitgestellten Federmechanismus 15, wie er später genauer beschrieben wird, als zumindest ein Teil des Auswurfmechanismus vorgesehen sein, und/oder kann zumindest ein Teil des Auswurfmechanismus dadurch bereitgestellt sein, dass die Umfangsabschnitte 19 der L-förmigen Schlitze 22 schräg verlaufen und als Führungskulisse dienen, wie vorstehend mit Referenz auf die Umfangsschlitze gemäß der zweiten Ausführungsform erläutert wurde. Der Federmechanismus 15 und die Führungskulisse können auch einen kombinierten Auswurfmechanismus bilden.

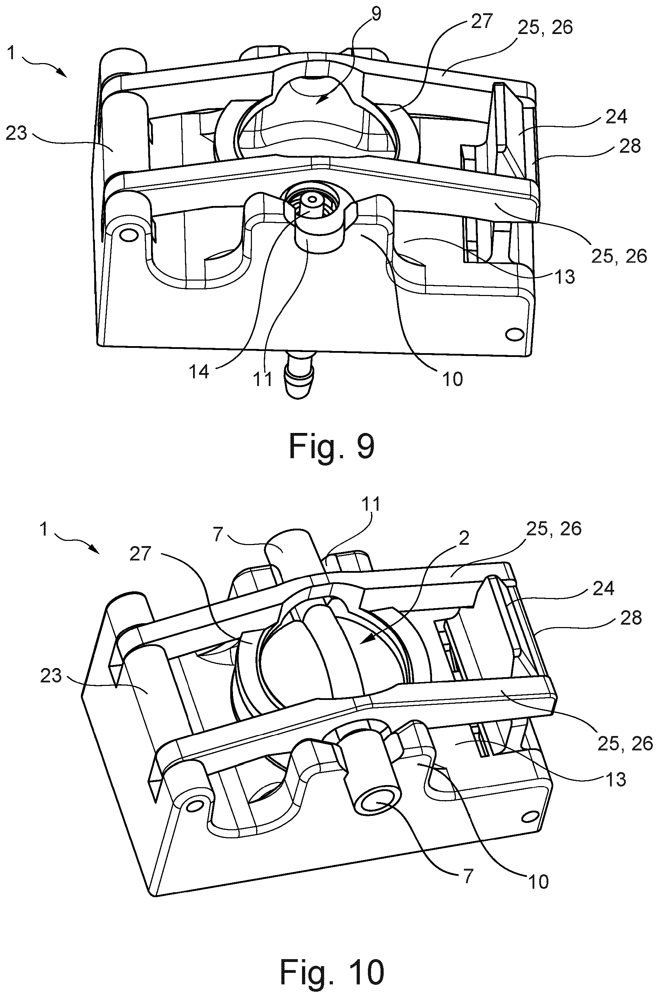

Fig. 9 und Fig. 10 zeigen eine vierte Ausführungsform der Halterung 1 der vorliegenden Erfindung mit und ohne eingesetzte Druckmesskapsel 2. Die Halterung 1 weist nach dieser Ausführungsform eine Basisplatte auf, welche den Boden 13 bildet und in/an welcher ein Federmechanismus 15 als Auswurfmechanismus, insbesondere ein später beschriebener Federmechanismus 15, sowie ein Halterungsanschluss 14 bereitgestellt ist, der mit dem Luftkammeranschluss 8 der Druckmesskapsel 2 kompatibel ist. In dieser Ausführungsform ist an einer Rückseite der Basisplatte ein ggf. steckbarer Anschluss zur schnellen Montage an einem Gehäuse bereitgestellt, wobei die Basisplatte dazu ausgebildet ist, in einem Montagezustand an dem Gehäuse anzuliegen. Ferner sind zwei einander gegenüberliegende, in diesem Beispiel jeweils außenseitig flach mit einem Rand der Basisplatte abschließende Wandabschnitte 10 bereitgestellt, welches sich senkrecht zu der Basisplatte bzw. in Einsetzrichtung oder Axialrichtung erstrecken. Die Wandabschnitte 10 bilden, wie bei den vorstehend beschriebenen Ausführungsformen, eine runde Innenumfangsfläche aus, die zur Aufnahme und Führung eines Außenumfangs der Druckmesskapsel 2 dient, und weisen zudem Axialschlitze 11 auf, die zur Aufnahme der Blutkammeranschlüsse 7 der Druckmesskapsel 2 dienen.

Winkelversetzt (um 90°) zu den einander gegenüberliegenden Wandabschnitten 10 sind einander ebenfalls gegenüberliegend, am Rand der Basisplatte nach vorne vorstehend, ein Scharnierwulst 23 und ein nach radial außen gerichteter, federnder Riegelhaken 24 vorgesehen. Der Scharnierwulst 23 bildet ein Scharnier mit einer parallel zu dem Boden 13 und dem Rand der Basisplatte verlaufenden Scharnierachse, um welche eine an dem Scharnier angelenkte Riegellasche 25 drehbar ist. Der federnde Riegelhaken 24, welcher dem Scharnierwulst 23 gegenüberliegt, bildet einen hier beispielhaft nach außen gerichteten Schnapphaken für einen Eingriff mit der Riegellasche 22.

Die Riegellasche 25 bildet im Wesentlichen einen am Scharnier befestigten Rahmen mit zwei Längsstreben 26, welche einen derartigen Knick aufweisen, dass die Riegellasche 25 im geschlossenen Zustand dachartig über dem Boden 13, insbesondere zentral über der Druckmesskapsel 2 liegt. Nahe des Knicks kann die Riegellasche 25 Mulden zur Aufnahme der Druckmesskapsel 2 sowie ein Anlagering 27 zur Anlage an der Kapsel 2 bereitstellen, um diese, wie in Fig. 10 gezeigt, in der Halterung 1 entgegen der Kraft des Federmechanismus 15 zu halten. Eine am freien Ende der Riegellasche 25 angeordnete Querstrebe bildet eine Eingriffskante 28 zum formschlüssigen Halten des Riegelhakens 24.

Um die Druckmesskapsel 2 in die Halterung 1 nach dieser Ausführungsform einzusetzen, wird zunächst die Riegellasche 25 geöffnet bzw. nach außen/vorne verschwenkt. Anschließend wird die Druckmesskapsel 2 derart in den Aufnahmeraum 9 eingesetzt, dass die Blutkammeranschlüsse 7 in den Axialschlitzen 11 liegen und der Luftkammeranschluss 8 und der Halterungsanschluss 14 miteinander verbunden werden. Daraufhin legt der Nutzer die Riegellasche 25 um, sodass diese, insbesondere mit dem Anlagering 27, auf dem Hauptkörper 3 der Druckmesskapsel 2 drückt, um die Kapsel gegen den Federmechanismus 15 zu drücken und eine gasdichte Verbindung zwischen dem Luftkammeranschluss und dem Halterungsanschluss 14 herzustellen. In einer Endlage wird die Querstrebe über den Riegelhaken 24 geschoben, sodass dieser ein-/umgreifend über die Eingriffskante 28 schnappt und die Riegellasche 25 verriegelt.

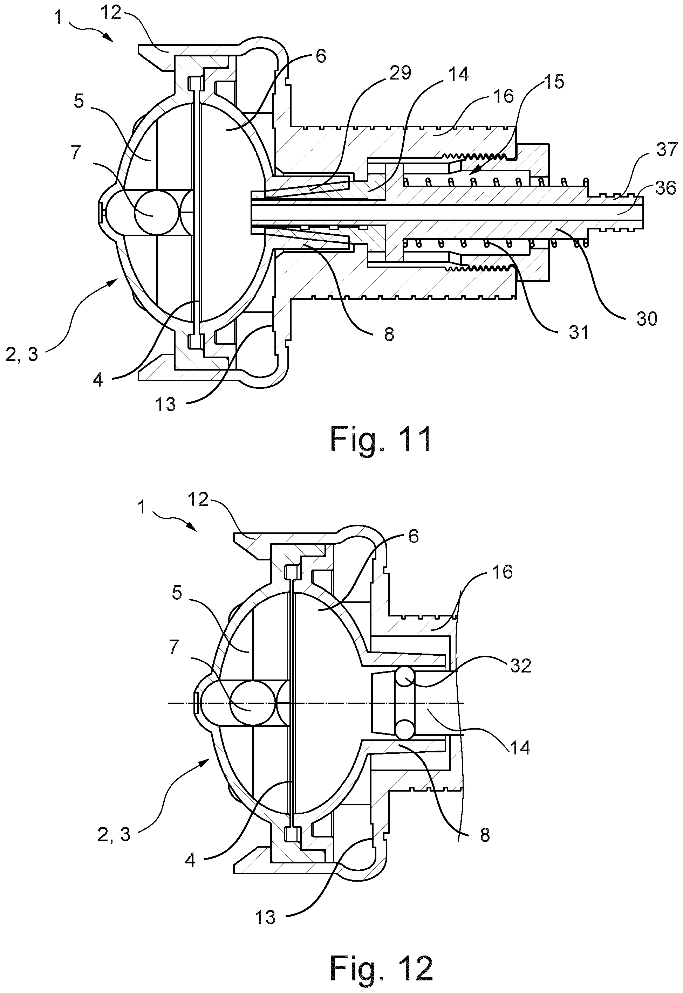

Fig. 11 zeigt die Druckmesskapselhalterung 1 nach der ersten Ausführungsform mit einer darin eingesetzten Druckmesskapsel 2 im Längsschnitt zur Veranschaulichung eines erfindungsgemäßen Federmechanismus 15 als dem Auswurfmechanismus oder als Teil desselben. Es ist verständlich, dass lediglich beispielhaft die erste Ausführungsform gewählt ist und dass der gleiche Federmechanismus 15 gleichermaßen in der modifizierten ersten und in der vierten Ausführungsform sowie ggf. auch in der zweiten und dritten Ausführungsform, jeweils in einem Boden 13, insbesondere Schaft 16, der entsprechenden Halterungen 1 angeordnet sein kann.

In der in Fig. 11 gezeigten Halterung sind die zwei einander diametral gegenüberliegenden Schnapphaken oder Federhaken 12 dargestellt, welche um einen Rand der in die Halterung eingesetzten Druckmesskapsel 2 greifen. Ferner ist der innere Aufbau Druckmesskapsel 2 mit der außen liegenden Blutkammer 5, der innen liegenden Luftkammer 6, der dazwischen angeordneten Membran 4 und einem der Blutkammeranschlüsse 7 erkennbar. An der Luftkammer 6 ist fluidleitend dazu ein Luftkammeranschluss 8 bereitgestellt, welcher in dem eingesetzten Zustand der Kapsel 2 in einen Schaft 16 der Halterung 1 hineinragt und welcher eine konische Innenfläche hat. Die konische Innenfläche sitzt auf einem einen Außenkonus bildenden Halterungsanschluss 14, welcher vorzugsweise mit einer Weichplastikschicht 29, insbesondere Silikonschicht, als einer Dichtung (zum Dichten zwischen dem Außenkonus und der konischen Innenfläche) versehen ist. Der Halterungsanschluss 14 ist auf einem mechanisch stabilen (verformungssteifen, insb. drucksteifen oder starren) vorzugsweise metallenen Druckstab 30 montiert und bildet zusammen mit diesem ein Druckelement. Der Druckstab 30 erstreckt sich durch den Schaft 16 und ist darin über eine Federanordnung 31, hier beispielhaft dargestellt eine Spiralfeder, axialverschiebbar gelagert. Die Federanordnung 31 und der Druckstab 30 bilden den Federmechanismus 15 als (ggf. teilweisen) Auswurfmechanismus. Der Druckstab 30 hat an seinem innenliegenden Ende, welches ggf. in das Gehäuse der extrakorporalen Blutbehandlungsmaschine hineinragt, einen Anschluss 37 z.B. für eine Verwendung mit einem Drucksensor. Ferner sind der Druckstab 30 und die Feder 31 in einer Hülse aufgenommen, die in den Schaft 16 eingeschraubt ist und somit leicht gelöst werden kann, z.B. zu Wartungszwecken.

Fig. 12, Fig. 13 und Fig. 14 zeigen ebenfalls die Druckmesskapselhalterung nach der ersten Ausführungsform im Längsschnitt mit weiteren Modifikationen und dienen der Veranschaulichung verschiedener Dichtungsanordnungen. Abgesehen von diesen Modifikationen ist davon auszugehen, dass die dargestellten Halterungen 1 im Wesentlichen gleich aufgebaut sind, weshalb nachfolgend lediglich Unterschiede erläutert werden. Beispielsweise ist es verständlich, dass, obwohl zur Vereinfachung in diesen Figuren kein Federmechanismus 15 dargestellt ist, dennoch ein solcher entsprechend Fig. 11 ausgebildeter Federmechanismus 15 darin entsprechend angeordnet ist oder angeordnet sein kann.

Wie in Fig. 12 dargestellt, kann alternativ zu der hier nicht dargestellten Weichplastikschicht 29 ein O-Ring 32 als eine Dichtung an dem Außenkonus des Halterungsanschlusses 14 in einer Außenumfangsnut aufgenommen sein, wodurch die Dichtung besser zu warten, insbesondere austauschbar ist. In diesem Fall kann vorzugsweise eine Kapsel 2 verwendet werden, bei welcher der Luftkammeranschluss 8 mit einer geradezylindrischen Innenfläche versehen ist, an welchem der O-Ring 32 anliegt, was einfacher zu fertigen ist und ferner eine höhere axiale Lagetoleranz ermöglicht. Ist ferner der Halterungsanschluss 14 an einem Druckstab 30 angebracht, d.h. an einem Federmechanismus 15 als (Teil eines) Auswurfmechanismus angeordnet, wirkt der Federmechanismus 15 beim Lösen der Greifvorrichtung, in diesem Beispiel der Schnapphaken, über die Reibkraft zwischen dem O-Ring und der geradezylindrischen Innenfläche des Luftkammeranschlusses 8 auf die Kapsel 2, um diese auszuwerfen. Ist bei der zweiten oder dritten Ausführungsform kein Federmechanismus 15, sondern nur eine Führungskulisse 18/22 als Auswurfmechanismus bereitgestellt, kann der Außenkonus auch starr in dem Schaft 16 angeordnet sein.

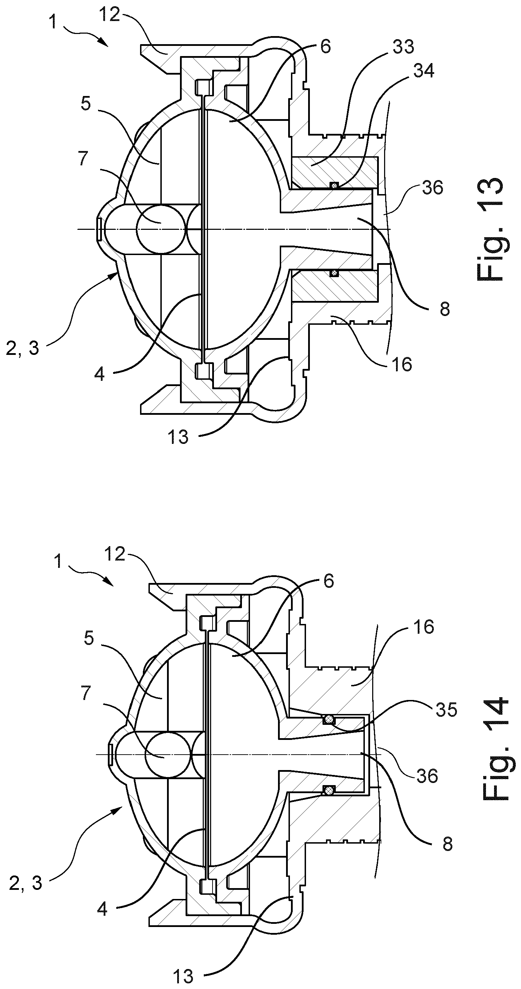

In Fig. 13 ist vorne (der eingesetzten Druckmesskapsel 2 zugewandt) an einem Eingang des Schafts 16 eine Trägerhülse 33 eingesetzt, in welcher eine Innenumfangsnut mit einem darin eingesetzten O-Ring 34 als Dichtung bereitgestellt ist, alternativ oder zusätzlich zu der vorstehend beschriebenen Weichplastikschicht 29 oder dem vorstehend beschriebenen, am Außenkonus angeordneten O-Ring 32. Der in Fig. 13 dargestellte O-Ring 34 liegt an einer geradezylindrischen Außenfläche des Luftkammeranschlusses 8 dichtend an und dichtet somit den Luftkammeranschluss 8 gegenüber dem Schaft 16 ab. Dies ist insbesondere dann von Vorteil, wenn der Schaft 16 selbst einen Teil einer Druckübertragungsleitung zwischen der Kapsel 2 und dem Drucksensor bildet und ist sowohl in Ausführungsformen mit als auch ohne Federmechanismus 15 als Auswurfmechanismus bereitstellbar. Die beanspruchte Erfindung beansprucht jedoch lediglich eine Druckmesskapselhalterung mit einem Federmechanismus 15 als Auswurfmechanismus.

In Fig. 14 ist im Unterschied zu Fig. 13 keine Trägerhülse 33 als Träger für den O-Ring 34 bereitgestellt, sondern ist ein O-Ring 35 in einer Außenumfangsnut an einer geradezylindrischen Außenfläche des Luftkammeranschlusses 8 bereitgestellt und liegt an einer geradezylindrischen Innenumfangsfläche des Schafts 16 dichtend an. Dies kann gemäß der vorstehend erläuterten Modifikation nach Fig. 13 in Ausführungsformen mit und ohne Federmechanismus 15 realisiert werden und insbesondere bei einem als Druckübertragungsleitung wirkenden Schaft 16 vorgesehen werden, ist gegenüber der Modifikation nach Fig. 13 jedoch einfacher zu fertigen und zu warten. Die beanspruchte Erfindung beansprucht jedoch lediglich eine Druckmesskapselhalterung mit einem Federmechanismus 15 als Auswurfmechanismus.

### Referenzzeichenliste

- 1: Druckmesskapselhalterung
- 2: Druckmesskapsel
- 3: Hauptkörper
- 4: Membran
- 5: Blutkammer
- 6: Luftkammer
- 7: Blutkammeranschluss
- 8: Fluid-/Luftkammeranschluss
- 9: Aufnahmeraum
- 10: Wand/Wandabschnitt
- 11: Schlitz/Axialschlitz
- 12: Schnapphaken/Federhaken (Greifvorrichtung)
- 13: Boden
- 14: (Halterungs-) Anschlusselement
- 15: Federmechanismus (Auswerfmechanismus)
- 16: Schaft
- 17: Fortsatz/Klammer (Greifvorrichtung)
- 18: Schlitz/Umfangsschlitz (Führungskulisse, Auswurfmechanismus)
- 19: Anschlussaufnahme
- 20: Rücksprung
- 21: Riegelring/Drehriegel (Greifvorrichtung)
- 22: L-förmiger Schlitz (Führungskulisse, Auswurfmechanismus)
- 23: Scharnierwulst/Scharnier (Greifvorrichtung)
- 24: Riegelhaken (Greifvorrichtung)
- 25: Riegellasche (Greifvorrichtung)
- 26: Längsstreben
- 27: Anlagering
- 28: Eingriffskante
- 29: Weichplastikschicht
- 30: Druckstab
- 31: Feder
- 32: O-Ring (Dichtungsmodifikation)
- 33: Trägerhülse
- 34: O-Ring (Dichtungsmodifikation)
- 35: O-Ring (Dichtungsmodifikation)
- 36: Druckübertragungsleitung
- 37: Anschluss

## Patentansprüche

1. Druckmesskapselhalterung (1), welche an einem Gehäuse einer extrakorporalen Blutbehandlungsmaschine, insbesondere einer Dialysemaschine, anbringbar ist, mit einer Greifvorrichtung (12; 17; 21; 23, 24, 25), die dazu angepasst ist, eine in die Druckmesskapselhalterung (1) einsetzbare Druckmesskapsel (2), die zumindest einen Blutkammeranschluss (7) hat und deren Innenraum durch eine Membran (4) in eine Blutkammer (5) und eine Fluidkammer (6) getrennt ist, um- oder eingreifend zu halten, und mit einer Druckübertragungsleitung (36), welche dazu konfiguriert ist, an die Fluidkammer (6) der Druckmesskapsel (2) fluidisch anschließbar zu sein und einen in der Fluidkammer (6) anliegenden Fluiddruck an einen Drucksensor der extrakorporalen Blutbehandlungsmaschine zu übertragen,
wobei die Druckmesskapselhalterung (1) einen Auswurfmechanismus (15; 18; 22) aufweist, welcher dazu angepasst ist, die Druckmesskapsel (2) bei einem Lösen der Greifvorrichtung (12; 17; 21; 23, 24, 25) auszuwerfen, und
wobei der Auswurfmechanismus (15; 18; 22) einen Federmechanismus (15) aufweist, mit einem Druckelement (30), welches derart federnd gelagert ist, dass es bei einem Einsetzen der Druckmesskapsel (2) vorgespannt wird und in einem Haltezustand, in welchem die Druckmesskapsel (2) durch die Greifvorrichtung (12; 17; 21; 23, 24, 25) gehalten wird, gegen diese presst,
wobei an einem äußeren Ende des Druckelements (30) ein die Druckübertragungsleitung (36) aufweisendes Halterungsanschlusselement (14) angeordnet oder ausgebildet ist.

2. Druckmesskapselhalterung (1) nach Anspruch 1, **dadurch gekennzeichnet, dass** die Greifvorrichtung (12; 17; 21; 23, 24, 25) über einen Schaft (16) an dem Gehäuse montierbar ist, wobei das Druckelement (30) in dem Schaft (16) aufgenommen und federnd gelagert ist.

3. Druckmesskapselhalterung (1) nach Anspruch 2, **dadurch gekennzeichnet, dass** das Halterungsanschlusselement (14) konisch, vorzugsweise aus einem Weichkunststoff, weiter vorzugsweise als ein Luer-Konnektor, ausgebildet ist.

4. Druckmesskapselhalterung (1) nach Anspruch 1, **dadurch gekennzeichnet, dass** eine Federkraft des Federmechanismus (15) eingestellt ist, um das Druckelement (30) im Haltezustand derart gegen die Druckmesskapsel (2) zu pressen, dass eine gasdichte Verbindung zwischen der Druckmesskapsel (2) und der Druckübertragungsleitung (36) bereitgestellt ist.

5. Druckmesskapselhalterung (1) nach einem der vorstehenden Ansprüche, **dadurch gekennzeichnet, dass** zumindest ein Wandabschnitt (10) bereitgestellt ist, welcher zumindest teilweise einen Aufnahmeraum (9) zur Aufnahme der Druckmesskapsel (2) definiert.

6. Druckmesskapselhalterung (1) nach Anspruch 5, **dadurch gekennzeichnet, dass** der zumindest eine Wandabschnitt (10) mit einem Schlitz (11; 18) zur Aufnahme des zumindest einen Blutkammeranschlusses (7) der Druckmesskapsel (2) bereitgestellt ist.

7. Druckmesskapselhalterung (1) nach Anspruch 5 oder 6, **dadurch gekennzeichnet, dass** eine Innenumfangsfläche des zumindest einen Wandabschnitts (10) ausgebildet ist, um als Führung für die Druckmesskapsel (2) zu dienen.

8. Druckmesskapselhalterung (1) nach einem der vorstehenden Ansprüche, **dadurch gekennzeichnet, dass** die Greifvorrichtung zumindest einen Schnapphaken (12) ausbildet, welcher dazu ausgebildet ist, bei einem Einsetzen der Druckmesskapsel (2) hinter die Druckmesskapsel (2) zu greifen.

9. Druckmesskapselhalterung (1) nach einem der Ansprüche 6 und 7, **dadurch gekennzeichnet, dass** der zumindest eine Schlitz ein quer zur Einsetzrichtung entlang des entsprechenden Wandabschnitts (10) verlaufender Umfangsschlitz (18) ist, durch welchen an einer Stirnseite des Wandabschnitts (10) der Druckmesskapselhalterung (1) die Greifvorrichtung als zumindest eine elastisch aufbiegbare, parallel zu dem Umfangsschlitz (18) verlaufende Klammer (17) abgeteilt oder ausgebildet ist, wobei der zumindest eine Blutkammeranschluss (7) durch ein Drehen der Druckmesskapsel (2) in den Umfangsschlitz (18) einsetzbar ist.

10. Druckmesskapselhalterung (1) nach Anspruch 9, **dadurch gekennzeichnet, dass** der zumindest eine Umfangsschlitz (18) an einem Ende verbreitert ist, um eine verrastbare Anschlussaufnahme (19) bereitzustellen.

11. Druckmesskapselhalterung (1) nach Anspruch 10, **dadurch gekennzeichnet, dass** an einem stirnseitigen Rand des zumindest einen Wandabschnitts (10), neben einer Ansatzstelle der Klammer (17), ein Rücksprung (20) vorgesehen ist.

12. Druckmesskapselhalterung nach einem der Ansprüche 6 und 7, **dadurch gekennzeichnet, dass** die Greifvorrichtung einen an einer Außenseite des zumindest einen Wandabschnitts (10) drehbar gelagerten, hülsenförmigen Drehriegel (21) aufweist, der zumindest einen L-förmigen Schlitz (22) hat, mit
einem Axialabschnitt, welcher sich entgegen einer Einsetzrichtung (9) an einer Stirnseite des Drehriegels (21) axial öffnet und welcher in einer Offenstellung des Drehriegels (21) den zumindest einen in dem Wandabschnitt bereitgestellten, axial verlaufenden Schlitz (11) derart überlagert, dass der zumindest eine Blutkammeranschluss (7) in den jeweiligen L-förmigen Schlitz (22) und axialen Schlitz (11) einsetzbar ist, und
einem Umfangsabschnitt, welcher ein in Umfangrichtung verlaufendes Schlitzende ausbildet und dazu angepasst ist, den zumindest einen Blutkammeranschluss (7) in einer durch Verdrehen des Drehriegels (21) erreichbaren Schließstellung zusammen mit dem axialen Schlitz (11) lagefestgelegt zu halten.

13. Druckmesskapselhalterung (1) nach einem der Ansprüche 9 bis 12,
**dadurch gekennzeichnet, dass** der Auswurfmechanismus eine Führungskulisse mit einem schräg zu einer Einsetzrichtung verlaufenden Schlitz, insbesondere dem Umfangsschlitz (18) oder dem Umfangsabschnitt des L-förmigen Schlitzes (22), aufweist.

14. Druckmesskapselhalterung (1) nach einem der Ansprüche 1 bis 7,
**dadurch gekennzeichnet, dass** die Greifvorrichtung eine über ein scharnierartiges Gelenk (23) angebrachte oder verschiebbar gelagerte Riegellasche (25) derart ausbildet, dass diese sich in einer Schließstellung an einer dem Federmechanismus (15) gegenüberliegenden Seite über die Druckmesskapsel (2) erstreckt, um diese gegen den Federmechanismus (15) zu drücken und zu halten.

## Claims

1. A pressure cell holder (1), which is attachable to a housing of an extracorporeal blood treatment machine, in particular a dialysis machine, with a gripping device (12; 17; 21; 23, 24, 25) adapted to grip around or engage a pressure cell (2) that is insertable into the pressure cell holder (1), which pressure cell has at least one blood chamber port (7) and whose interior space is separated by a membrane (4) into a blood chamber (5) and a fluid chamber (6), and with a pressure transmission line (36), which is configured to be fluidically connectable to the fluid chamber (6) of the pressure cell (2) and to transmit a fluid pressure present in the fluid chamber (6) to a pressure sensor of the extracorporeal blood treatment machine,
the pressure cell holder (1) having an ejection mechanism (15; 18; 22) adapted to eject the pressure cell (2) upon release of the gripping device (12; 17; 21; 23, 24, 25), and
the ejection mechanism (15; 18; 22) comprising a spring mechanism (15) having a pressure element (30) which is spring-mounted in such a way that it is pre-tensioned upon insertion of the pressure cell (2) and presses against the pressure cell (2) in a holding state in which the pressure cell (2) is held by the gripping device (12; 17; 21; 23, 24, 25),
wherein at an outer end of the pressure element (30), a holder port element (14) comprising the pressure transmission line (36) is arranged or formed.

2. The pressure cell holder (1) according to claim 1, **characterized in that** the gripping device (12; 17; 21; 23, 24, 25) is mountable on the housing via a shaft (16), the pressure element (30) being received in the shaft (16) and being spring-mounted.

3. The pressure cell holder (1) according to claim 2 or 3, **characterized in that** the holder port element (14) is conical, preferably made of a soft plastic, further preferably formed as a Luer connector.

4. The pressure cell holder (1) according to claim 1, **characterized in that** a spring force of the spring mechanism (15) is set to press the pressure element (30) in the holding state against the pressure cell (2) in such a way that a gas-tight connection is provided between the pressure cell (2) and the pressure transmission line (36).

5. The pressure cell holder (1) according to any of the preceding claims, **characterized in that** at least one wall portion (10) is provided which at least partially defines a receiving chamber (9) for receiving the pressure cell (2).

6. The pressure cell holder (1) according to claim 5, **characterized in that** the at least one wall portion (10) is provided with a slit (11; 18) for receiving the at least one blood chamber port (7) of the pressure cell (2).

7. The pressure cell holder (1) according to claim 5 or 6, **characterized in that** an inner circumferential surface of the at least one wall portion (10) is formed to serve as a guide for the pressure cell (2).

8. The pressure cell holder (1) according to any of the preceding claims, **characterized in that** the gripping device forms at least one snap-fit hook (12) which is designed to engage behind the pressure cell (2) when the pressure cell (2) is inserted.

9. The pressure cell holder (1) according to any of claims 6 and 7, **characterized in that** the at least one slit is a circumferential slit (18) extending transversely to the insertion direction along the corresponding wall portion (10), through which the gripping device is separated or formed as at least one elastically bendable clamp (17) extending parallel to the circumferential slit (18) at one front end of the wall portion (10) of the pressure cell holder (1), wherein the at least one blood chamber port (7) is insertable into the circumferential slit (18) by rotating the pressure cell (2).

10. The pressure cell holder (1) according to claim 10, **characterized in that** the at least one circumferential slit (18) is widened at one end to provide a latchable port receptacle (19).

11. The pressure cell holder (1) according to claim 10, **characterized in that** a setback (20) is provided at a front edge of the at least one wall portion (10), next to an attachment point of the clamp (17).

12. The pressure cell holder according to any of claims 6 and 7, **characterized in that** the gripping device comprises a sleeve-shaped turning bolt (21) rotatably mounted on an outer side of the at least one wall portion (10) and having at least one L-shaped slit (22), having
an axial portion which opens axially against an insertion direction (9) on a front face of the turning bolt (21) and which, in an open position of the turning bolt (21), overlaps the at least one axially extending slit (11) provided in the wall portion in such a way that the at least one blood chamber port (7) is insertable into the respective L-shaped slit (22) and axial slit (11), and
a circumferential portion which forms a circumferentially extending slit end and is adapted to hold the at least one blood chamber port (7) in a fixed position together with the axial slit (11) in a closed position achievable by turning the turning bolt (21).

13. The pressure cell holder (1) according to any of claims 9 to 12, **characterized in that** the ejection mechanism has a guide link with a slit running obliquely to an insertion direction, in particular to the circumferential slit (18) or to the circumferential portion of the L-shaped slit (22).

14. The pressure cell holder (1) according to any of claims 1 to 7, **characterized in that** the gripping device forms a locking tab (25) attached or displaceably mounted via a hinge-like joint (23) in such a way that, in a closed position, the locking tab extends over the pressure cell (2) on a side opposite the spring mechanism (15) in order to press and hold the pressure cell (2) against the spring mechanism (15).

## Revendications

1. Support pour capsule de mesure de pression (1) qui peut être fixé sur un boîtier d'une machine de traitement du sang extracorporel, en particulier d'une machine de dialyse, avec un dispositif de préhension (12 ; 17 ; 21 ; 23, 24, 25) qui est adapté pour maintenir de manière enveloppante ou en prise une capsule de mesure de pression (2) pouvant être insérée dans le support pour capsule de mesure de pression (1) qui présente au moins un raccord de chambre à sang (7) et dont l'espace intérieur est séparé par une membrane (4) en une chambre pour le sang (5) et une chambre pour le fluide (6), et avec une conduite de transmission de pression (36) qui est configurée pour pouvoir être raccordée par voie fluidique à la chambre pour le fluide (6) de la capsule de mesure de pression (2) et pour transmettre une pression de fluide régnant dans la chambre pour le fluide (6) à un capteur de pression de la machine de traitement du sang extracorporel,
dans lequel le support pour capsule de mesure de pression (1) présente un mécanisme d'éjection (15 ; 18 ; 22) qui est adapté pour éjecter la capsule de mesure de pression (2) lors d'un desserrage du dispositif de préhension (12 ; 17 ; 21 ; 23, 24, 25), et
dans lequel le mécanisme d'éjection (15 ; 18 ; 22) présente un mécanisme à ressort (15) avec un élément de pression (30) qui est monté de manière élastique de sorte qu'il est précontraint lors de l'insertion de la capsule de mesure de pression (2) et appuie contre celle-ci dans un état de maintien dans lequel la capsule de mesure de pression (2) est maintenue par le dispositif de préhension (12 ; 17 ; 21 ; 23, 24, 25),
dans lequel un élément de raccordement de support (14) présentant la conduite de transmission de pression (36) est disposé ou formé à une extrémité extérieure de l'élément de pression (30).

2. Support pour capsule de mesure de pression (1) selon la revendication 1, **caractérisé en ce que** le dispositif de préhension (12 ; 17 ; 21 ; 23, 24, 25) peut être monté sur le boîtier par l'intermédiaire d'une tige (16), dans lequel l'élément de pression (30) est logé dans la tige (16) et monté de manière élastique.

3. Support pour capsule de mesure de pression (1) selon la revendication 2, **caractérisé en ce que** l'élément de raccordement de support (14) est conçu de manière conique, de préférence en un plastique souple, de préférence en outre comme un connecteur Luer.

4. Support pour capsule de mesure de pression (1) selon la revendication 1, **caractérisé en ce qu'**une force élastique du mécanisme à ressort (15) est réglée pour presser l'élément de pression (30) contre la capsule de mesure de pression (2) à l'état de maintien, de sorte qu'une connexion étanche au gaz est établie entre la capsule de mesure de pression (2) et la conduite de transmission de pression (36).

5. Support pour capsule de mesure de pression (1) selon l'une quelconque des revendications précédentes, **caractérisé en ce qu'**au moins une section de paroi (10) est fournie, laquelle définit au moins partiellement un espace de logement (9) pour loger la capsule de mesure de pression (2).

6. Support pour capsule de mesure de pression (1) selon la revendication 5, **caractérisé en ce que** la au moins une section de paroi (10) est munie d'une fente (11 ; 18) destinée à loger le au moins un raccord de chambre pour le sang (7) de la capsule de mesure de pression (2).

7. Support pour capsule de mesure de pression (1) selon la revendication 5 ou 6, **caractérisé en ce qu'**une surface périphérique intérieure de la au moins une section de paroi (10) est formée pour servir de guide à la capsule de mesure de pression (2).

8. Support pour capsule de mesure de pression (1) selon l'une quelconque des revendications précédentes, **caractérisé en ce que** le dispositif de préhension forme au moins un crochet d'encliquetage (12) qui est conçu pour s'accrocher derrière la capsule de mesure de pression (2) lors de l'insertion de la capsule de mesure de pression (2).

9. Support pour capsule de mesure de pression (1) selon l'une quelconque des revendications 6 et 7, **caractérisé en ce que** la au moins une fente est une fente périphérique (18) s'étendant transversalement à la direction d'insertion le long de la section de paroi correspondante (10), fente à travers laquelle le dispositif de préhension est séparé ou conçu sur une face avant de la section de paroi (10) du support pour capsule de mesure de pression (1) en tant qu'au moins une pince élastiquement pliable (17) s'étendant parallèlement à la fente périphérique (18), dans lequel le au moins un raccord de chambre pour le sang (7) peut être inséré dans la fente périphérique (18) par une rotation de la capsule de mesure de pression (2).

10. Support pour capsule de mesure de pression (1) selon la revendication 10, **caractérisé en ce que** la au moins une fente périphérique (18) est élargie à une extrémité pour fournir un logement de raccordement encliquetable (19).

11. Support pour capsule de mesure de pression (1) selon la revendication 10, **caractérisé en ce qu'**un retrait (20) est prévu sur un bord avant de la au moins une section de paroi (10), à côté d'un point d'attache de la pince (17).

12. Support pour capsule de mesure de pression selon l'une quelconque des revendications 6 et 7, **caractérisé en ce que** le dispositif de préhension présente un verrou rotatif (21) en forme de douille, monté de manière rotative sur un côté extérieur de la au moins une section de paroi (10), verrou qui présente au moins une fente en forme de L (22), avec
une section axiale qui s'ouvre axialement à l'encontre d'une direction d'insertion (9) sur un côté avant du verrou rotatif (21) et qui, dans une position ouverte du verrou rotatif (21), recouvre la au moins une fente (11) s'étendant axialement fournie dans la section de paroi de sorte que le au moins un raccord de chambre pour le sang (7) peut être inséré dans la fente en forme de L (22) et la fente axiale (11) respectives, et
une section périphérique qui forme une extrémité de fente s'étendant dans la direction périphérique et est adaptée pour maintenir le au moins un raccord de chambre pour le sang (7) dans une position de fermeture pouvant être atteinte par rotation du verrou rotatif (21) en position fixe avec la fente axiale (11).

13. Support pour capsule de mesure de pression (1) selon l'une quelconque des revendications 9 à 12, **caractérisé en ce que** le mécanisme d'éjection présente une coulisse de guidage avec une fente s'étendant obliquement par rapport à une direction d'insertion, en particulier la fente périphérique (18) ou la section périphérique de la fente en forme de L (22).

14. Support pour capsule de mesure de pression (1) selon l'une quelconque des revendications 1 à 7, **caractérisé en ce que** le dispositif de préhension forme une languette de verrouillage (25) montée ou posée de manière coulissante sur une articulation de type charnière (23) de sorte que, dans une position de fermeture, celle-ci s'étend sur la capsule de mesure de pression (2) sur un côté opposé au mécanisme à ressort (15) pour presser et maintenir celle-ci contre le mécanisme à ressort (15).
